# EUROPEAN PATENT APPLICATION

(11) **EP 4 245 317 A1**
(43) Date of publication of application: **20.09.2023**
(21) Application number: 21891133.7
(22) Date of filing: 10.11.2021
(51) Int. Cl.: A61K 39/395, A61P 35/00, C07K 16/28, C07K 16/30

(54) **BISPECIFIC ANTIBODY FOR CLAUDIN 18A2 AND CD3 AND APPLICATION OF BISPECIFIC ANTIBODY**

(30) Priority: 10.11.2020 CN 202011249960
(71) Applicant: Shanghai Qilu Pharmaceutical Research and Development Centre Ltd., Shanghai 201203 (CN)
(72) Inventor: JIANG, Jiahua, Shanghai 201203 (CN); LUO, Xiao, Shanghai 201203 (CN); GU, Jinming, Shanghai 201203 (CN); CHOU, Chuan-Chu, Shanghai 201203 (CN); CHEN, Shihao, Shanghai 201203 (CN); YANG, Yingying, Shanghai 201203 (CN)
(74) Representative: Elzaburu S.L.P.
(86) International application number: PCT/CN2021/129778
(87) International publication number: WO 2022/100613

(57) **Abstract**

The present disclosure relates to a bispecific antibody directed against Claudin 18.2 and CD3, pharmaceutical compositions comprising said bispecific antibody, and related use thereof in the treatment of cancer.

## Description

### Technical Field

The present disclosure is in the field of immunology, and more particularly, the present disclosure relates to a bispecific antibody against Claudin 18.2 (CLDN18A2, CLDN18.2) and CD3. The antibody contains both a binding domain that recognizes Claudin 18.2 and a CD3 binding domain that specifically binds to T cells. Also, the present disclosure relates to pharmaceutical composition comprising said bispecific antibody and related use thereof in the treatment of cancer.

### Background Art

Claudin 18 (CLDN18) is an integral membrane protein located in the tight junctions of epithelium and endothelium, with a molecular weight of about 27.9 KD. CLDN18 forms intercellular tight junctions with other tight junctions, regulating the permeability of tissue molecules and ions in the intercellular space, and maintaining the stability of the tissue environment. Take Claudin 18 in human for example, which has two subtypes, splice variant 1 (CLDN 18A1, CLDN18.1): GenBank Accession Numbers NP_057453, NM_016369, and splice variant 2 (CLDN 18A2, CLDN18.2): GenBank Accession Numbers NP 001002026, NM 001002026. In normal cells, CLDN18A1 is selectively expressed in the epithelial cell of the lung, while CLDN18A2 is specifically expressed in normal gastric epithelial differentiated cells and not expressed in gastric epithelial stem cells with cell division activity. However, CLDN18A2 is overexpressed in tumor cells in various cancer types, such as high expression of CLDN18A2 found in 75% of gastric cancer patients, high expression of CLDN18A2 found in 50% of pancreatic cancer patients, and high expression of CLDN18A2 found in 30% of esophageal cancer patients, also in lung cancer and other cancer types. Therefore, finding antibodies that specifically bind to CLDN18A2 but not CLDN18A1 is of great significance for the treatment and detection of cancer.

The existing CLDN18.2 antibody IMAB362 has entered the clinical research stage, clinical results showed that in patients with gastric cancer with high expression of CLDN18.2, compared with chemotherapy alone, the progression-free survival time of chemotherapy + IMAB362 was extended from 6.1 months to 9.1 months, and the total survival time was extended from 9.3 months to 16.6 months. In addition to the antibody IMAB362, at least three CLDN18.2 monoclonal antibody drugs are entering Phase I clinical study currently. CAR-T prepared against CLDN18.2 targets has also entered clinical studies. However, these antibodies that have entered clinical stage have weak affinity against CLDN18.2, and have weak preclinical anti-tumor effect in vivo and large side effects. Therefore, there is still a need to continue to screen and prepare CLDN18.2 antibodies with higher activity, lower toxicity, and a larger therapeutic safety window, so as to produce a stronger pharmacodynamic effect at a smaller dosage and to improve the optimal dosing space.

T cell-based therapies have shown significant anti-tumor effects in a number of animal models, and a number of T cell therapies have recently made significant progress in the treatment of cancer indications. Therefore, it is very important to develop a novel T cell bispecific antibody with high efficiency and low toxicity by exerting the potential of T cells. The present disclosure provides a bispecific antibody against CLDN18.2 and CD3, which is capable of recruiting T cells to tumor sites through a CD3 target, specifically killing tumor cells highly expressing CLDN18.2, making it possible to target the cytotoxic effect of T cells to cancer cells. At present, the bispecific binding molecules known in the prior art which simultaneously recognize CLDN18.2 and CD3ε, such as 1BiMAB disclosed in CN105073776 B, have a relatively small molecular weight and also lack components such as Fc fragment, therefore the molecule has a relatively short half-life and poor stability. Thus, there remains a need to construct a bispecific binding molecule with improved in vivo and in vitro stability and a longer half-life, such that once- or twice-weekly intravenous administration is expected, rather than daily intravenous administration as required for 1BiMAB.

### Summary of the Invention

The present disclosure provides a bispecific antibody comprising anti-CLDN18.2 binding domain and anti-CD3 binding domain, the first binding domain being capable of binding to CLDN18.2 protein and the second binding domain being capable of binding to CD3ε. In some embodiments, the CLDN18.2 is a protein having GenBank accession number NP_001002026 (mRNA: NM_001002026). CLDN18.1 is a protein having GenBank accession number NP_057453 (mRNA: NM_016369).

In the bispecific antibody of the present disclosure, the anti-CLDN18.2 binding domain is derived from antibody 6#AA, the antibody 6#AA or an antigen-binding fragment thereof specifically binds to CLDN18.2 but does not significantly bind to CLDN 18.1. In some embodiments, the binding level of antibody 6#AA or an antigen binding fragment thereof binding to CLDN18.1 is no more than 20% of that binding to CLDN18.2. For example, the binding level can be 20%, 19%, 18%, 17%, 16%, 15%, 14%, 13%, 12%, 11%, 10%, 9%, 8%, 7%, 6%, 5%, 4%, 3%, 2%, 1%, or less than 1% of that of the antibody or an antigen-binding fragment thereof binding to CLDN18.2. In some embodiments, the binding level of antibody 6#AA or an antigen-binding fragment thereof binding to CLDN18.2 is 1-fold, 2-fold, 3-fold, 4-fold, 5-fold, 6-fold, 7-fold, 8-fold, 9-fold, 10-fold, or more than 10-fold greater than that of binding to CLDN18.1.

The antibody 6#AA or an antigen-binding fragment thereof is capable of specifically binding to CLDN18.2, comprising: a heavy chain variable region in which the sequences of three CDRs, i.e., HCDR1, HCDR2, and HCDR3, are as set forth in SEQ ID NO: 11, 12, and 13, respectively; and a light chain variable region in which the sequences of three CDRs, i.e., LCDR1, LCDR2, and LCDR3, are as set forth in SEQ ID NO: 14, 15, and 16, respectively.

Thus, in the bispecific antibody of the present disclosure, the anti-CLDN18.2 binding domain is also capable of specifically binding to CLDN18.2, comprising: a heavy chain variable region in which the sequences of three CDRs, i.e., HCDR1, HCDR2, and HCDR3, are as set forth in SEQ ID NO: 11, 12, and 13, respectively; and a light chain variable region in which the sequences of three CDRs, i.e., LCDR1, LCDR2, and LCDR3, are as set forth in SEQ ID NO: 14, 15, and 16, respectively.

The antibody 6#AA or an antigen-binding fragment thereof comprises a heavy chain variable region having at least 80% to 100% sequence identity to SEQ ID NO: 23 and a light chain variable region having at least 80% to 100% sequence identity to SEQ ID NO: 24.

Thus, in the bispecific antibody of the present disclosure, the anti-CLDN18.2 binding domain also comprises a heavy chain variable region and a light chain variable region, the heavy chain variable region has at least 80% to 100% sequence identity to SEQ ID NO: 23; and the light chain variable region has at least 80% to 100% sequence identity to SEQ ID NO: 24.

In the bispecific antibody of the present disclosure, the anti-CD3 binding domain is derived from antibody h160C9AA, the antibody h160C9AA or an antigen-binding fragment thereof specifically binds to CD3. The h160C9AA comprises a heavy chain variable region in which the sequences of three CDRs, i.e., HCDR1, HCDR2, and HCDR3, are as set forth in SEQ ID NO: 17, 18, and 19, respectively; and a light chain variable region in which the sequences of three CDRs, i.e., LCDR1, LCDR2, and LCDR3, are as set forth in SEQ ID NO: 20, 21, and 22, respectively.

Thus, in the bispecific antibody of the present disclosure, the anti-CD3 binding domain is also capable of specifically binding to CD3, comprising: a heavy chain variable region, in the heavy chain variable region the sequences of three CDRs, i.e., HCDR1, HCDR2, and HCDR3, are as set forth in SEQ ID NO: 17, 18, and 19, respectively; and a light chain variable region, in the light chain variable region the sequences of three CDRs, i.e., LCDR1, LCDR2, and LCDR3, are as set forth in SEQ ID NO: 20, 21, and 22, respectively.

The antibody h160C9AA or an antigen-binding fragment thereof comprises a heavy chain variable region and a light chain variable region, the heavy chain variable region has at least 80% to 100% sequence identity to SEQ ID NO: 6; and the light chain variable region has at least 80% to 100% sequence identity to SEQ ID NO: 7.

Thus, in the bispecific antibody of the present disclosure, the anti-CD3 binding domain also comprises a heavy chain variable region and a light chain variable region, the heavy chain variable region has at least 80% to 100% sequence identity to SEQ ID NO: 6; and the light chain variable region has at least 80% to 100% sequence identity to SEQ ID NO: 7.

In some preferred embodiments, the bispecific antibody of the present disclosure comprises a binding domain which is Fab, Fv, scFv, F(ab')₂, a linear antibody, a single domain antibody, or a full-length antibody.

In some preferred embodiments, the bispecific antibody of the present disclosure further comprises a heavy chain constant region and/or a light chain constant region, preferably the heavy chain constant region comprises an Fc or a variant Fc, preferably the Fc is derived from human.

In some more preferred embodiments, the bispecific antibody with structures constructed by linking a CD3-binding scFv at the C-terminus of one heavy chain of the anti-CLDN18.2 full-length antibody or by linking a CD3-binding scFv at the C-terminus of two light chains of the anti-CLDN18.2 full-length antibody, so that two bispecific antibodies with different structures are constructed. The bispecific antibody molecules are capable of recruiting T cells to a target tumor site by binding to the CD3 antigen, while achieving specific killing by recognizing tumor cells with high expression of CLDN18.2. In some preferred embodiments, the anti-CLDN18.2 binding domain in the bispecific antibody of the present disclosure is a full-length antibody, the anti-CD3 binding domain is scFv. The heavy chain sequence of the full-length antibody is as set forth in SEQ ID NO: 1, the light chain sequence is as set forth in SEQ ID NO: 5. scFv is constructed by connecting VH and VL by a linker, either VH-linker-VL or VL-linker-VH, preferably VL-linker-VH, which has a sequence as set forth in SEQ ID NO: 8.

In a preferred embodiment, the bispecific antibody of the present disclosure is formed by linking anti-CD3 scFv to the C-terminus of one heavy chain or to the C-terminus of both light chains of an anti-CLDN18.2 full-length antibody.

In a preferred embodiment, the bispecific antibody formed by fusing each of the two anti-CD3 scFv peptide chains to the C-terminus of the two light chains of an anti-CLDN18.2 full-length antibody comprises the two fused homologous light chains and the two unaltered homologous heavy chains, wherein the fused light chain has a sequence as set forth in SEQ ID NO: 2 and the heavy chain has a sequence as set forth in SEQ ID NO: 1.

In a preferred embodiment, the bispecific antibody formed by fusing one anti-CD3 scFv peptide chain to the C-terminus of a heavy chain of an anti-CLDN18.2 full-length antibody comprises two homologous light chains and two heterologous heavy chains. Among the two heterologous heavy chains, the heavy chain containing scFv is constructed as a "knob" structure after several amino acid substitutions, and the heavy chain without scFv is constructed as a "hole" structure after several amino acid substitutions. Wherein the constructed homologous light chain has a sequence as set forth in SEQ ID NO: 5, the heavy chain with a "knob" structure has a sequence as set forth in SEQ ID NO: 3, the heavy chain with a "hole" structure has a sequence as set forth in SEQ ID NO: 4.

The present disclosure also provides a nucleic acid encoding the bispecific antibody; and a recombinant vector comprising the nucleic acids, preferably the vector is a recombinant expression vector.

In some embodiments, the present disclosure also provides a host cell comprising the recombinant vector, or genome of which integrated with the nucleic acid encoding the bispecific antibody. In some preferred embodiments, the host cell may be a prokaryotic cell, such as E. coli; may also be eukaryotic cells such as yeast or mammalian cells such as CHO cells, HEK293 cells, HEK293E cells, or Expi293 cells.

In some embodiments, the present disclosure provides a method of preparing the bispecific antibody, comprising: culturing the host cells of the present disclosure under suitable conditions and purifying the expression products from the cells.

In some embodiments, the present disclosure provides the use of the bispecific antibody for the preparation of a drug that specifically targets CLDN18.2-expressing tumor cells; in some embodiments, the CLDN18.2-expressing tumor comprises: gastric cancer, pancreatic cancer, esophageal cancer, lung cancer, ovarian cancer, colon cancer, rectal cancer, liver cancer, head and neck cancer, and gallbladder cancer and metastases thereof, the gastric cancer metastasis such as Kuckenberg tumor.

In some embodiments, the present disclosure provides a pharmaceutical composition comprising an effective amount of bispecific antibody of the present disclosure or comprising an effective amount of nucleic acids encoding the bispecific antibody, or comprising an effective amount of a recombinant vector containing an encoding nucleic acid, or comprising an effective amount of a host cell comprising an encoding nucleic acid. In some embodiments, the pharmaceutical composition further comprises a pharmaceutically acceptable carrier.

In some preferred embodiments, the pharmaceutical composition further comprises one or more additional other therapeutic agents. The additional therapeutic agents include: cytotoxic agents, cytostatic agents, anti-angiogenic agents, anti-neoplastic agents, chemotherapeutic agents, radio therapeutic agents, targeted anti-cancer agents, biological response modifiers, cancer vaccines, cytokines, hormones, anti-metastatic agents, and immunotherapeutic agents.

In some embodiments, the present disclosure provides a drug box or kit comprising a container, and a pharmaceutical composition of the present disclosure in the container.

In some embodiments, the present disclosure provides a method of inducing death in CLDN18.2-expressing cells, comprising contacting the cells with the pharmaceutical composition of the present disclosure. In some embodiments, the cells are contacted with the pharmaceutical composition in vitro. In some embodiments, the cells are contacted with the pharmaceutical composition in vivo. In some embodiments, the cells are tumor cells. In some embodiments, the cells are solid tumor cells. In some embodiments, the cells are selected from the group consisting of: gastric cancer cells, esophageal cancer cells, intestinal cancer cells, pancreatic cancer cells, nephroblastoma cells, lung cancer cells, ovarian cancer cells, colon cancer cells, rectal cancer cells, liver cancer cells, head and neck cancer cells, chronic myelogenous leukemia cells, and gallbladder cancer cells. In some embodiments, the present disclosure provides a method of treating a disease associated with expression of CLDN18.2 in a subject, comprising administering to a subject in need thereof a pharmaceutical composition of the present disclosure. In some embodiments, the disease is a tumor. In some embodiments, the tumor is preferably gastric cancer, esophageal cancer, intestinal cancer, pancreatic cancer, nephroblastoma, lung cancer, ovarian cancer, colon cancer, rectal cancer, liver cancer, head and neck cancer, chronic myelogenous leukemia, or gallbladder cancer. In some embodiments, the method further comprises administering to the subject one or more additional therapeutic agents. The antibody of the present disclosure may be administered in combination with one or more additional therapeutic agents, including, but not limited to, chemotherapeutic agents, cytotoxic agents, radio therapeutic agents, cancer vaccines, anti-neoplastic agents, targeted anti-cancer agents, anti-angiogenic agents, biological response modifiers, cytokines, hormones, anti-metastatic agents, and immunotherapeutic agents.

In some preferred embodiments, the chemotherapeutic agents that can be used in combination with an antibody or antigen-binding fragment thereof of the present disclosure include, but are not limited to, mitotic inhibitors, including vincristine, vinblastine, vindesine, and navelbine; topoisomerase I inhibitors, such as camptothecin compounds, including irinotecan, topotecan and other compounds derived from camptothecin and analogs thereof; podophyllotoxin derivatives such as etoposide, teniposide and midoxizoz; alkylating agents such as cisplatin, carboplatin, cyclophosphamide, nitrogen mustard, trimethylenethiophosphoramide, carmustine, busulfan, chlorambucil, briquinolizine, uracil mustard, cloprofen and dacarbazine; antimetabolites, including cytarabine, 5-fluorouracil, methotrexate, mercaptopurine, azathioprine, and procarbazine; antibiotics including, but not limited to, doxorubicin, bleomycin, dactinomycin, daunorubicin, mitomycin, sarcomycin C, actinomycin D, roxithromycin, doxorubicin, rapamycin and derivatives thereof, and daunomycin; and other chemotherapeutic agents including, but not limited to, paclitaxel, docetaxel, dacarbazine, azacytidine, amsacon, melphalan, ifosfamide, and mitoxantrone. In some preferred embodiments, the one or more additional therapeutic agents are selected from one or more of epirubicin, oxaliplatin, and 5-fluorouracil.

In some embodiments, the targeted anticancer agents include, but are not limited to, large molecule targeted drugs, small molecule targeted drugs, etc.

In some preferred embodiments, the macromolecular targeting agents include, but are not limited to, epidermal growth factor targeting agents, including cetuximab, panitumumab, and nimotuzumab, etc.; HER-2 or HER-3 signaling pathway inhibitors, including trastuzumab, pertuzumab, T-DM1, etc.; anti-vascular endothelial growth factor drugs, including VEGF-TRAP, bevacizumab, ramucirumab, etc.; also, agents targeting other targets include, but are not limited to, targets such as PI3K, PARP, PI3Kα, PKB/AKT, and STAT3.

In some embodiments, small molecule targeting agents include, but are not limited to, epidermal growth factor targeting agents, including erlotinib or gefitinib, etc.; HER-2 or HER-3 signaling pathway inhibitors, including lapatinib or afatinib, etc.; tyrosine kinase inhibitors including imatinib or sunitinib, etc.; anti-vascular endothelial growth factor drugs including sorafenib, regorafenib, pazopanib, recombinant human endostatin, apatinib, etc.; targeting c-Met/ROS1 drugs, including crizotinib, etc.; and, other targeting agents, including but not limited to vorinostat and marimastat, etc.; targeting mTOR drugs, including everolimus, etc.; and agents targeting other targets including but not limited to PI3Kα, PKB/AKT and STAT3.

In some embodiments, the immunotherapeutic agents include, but are not limited to, immunosuppressive agents and agonists, wherein the targets include PD-1/PD-L1, PD-L2, CTLA-4, LAG-3, IDO, TIM3, TIGIT, CD47, SIRPα, 4-1BB, CSF-1/CSF1R, GITR, OX40, CD40, CD27, CD28, B7H4, B7H3, TGFβ, BTLA, VISTA, ICOS, CD39, CD73, A2AR, KIR, and NKG2A, etc.; and cell therapy associated with immunotherapy.

In some embodiments, immune checkpoint inhibitors that target PD-1/PD-L1 include, but are not limited to, macromolecular drugs such as, pembrolizumab, nivolumab, atezolizumab, avelumab, and sintilimab, Cmiplimab, and Durvomab, etc.; and small molecule drugs.

In some embodiments, immune checkpoint inhibitors that target CTLA-4 include, but are not limited to, ipilimumab, etc.; cytokines include, but are not limited to, IL-10, IL-15, IL4, and IL13, etc.; inhibitors that target BRAF include, but are not limited to, Binimetinib, etc. In some embodiments, the other therapeutic agent is selected from oncolytic viruses, such as parvovirus, adenovirus, herpes virus, poxvirus, poliovirus, reovirus, alphavirus, malaba virus, retrovirus, and coxsackie virus, etc.; alternatively, the other therapeutic agent is selected from cancer vaccines or protease inhibitors, such as bortezomib, etc.

### Brief Description of the Drawings

The drawings further illustrate the novel features disclosed in this specification. The features and advantages disclosed in this specification will be better understood with reference to the drawings, but it is to be understood that these drawings are merely illustrative of specific embodiments of the principles disclosed herein and are not intended to limit the scope of the appended claims.
FIG. 1 shows the structures of two bispecific antibodies of the present disclosure.
FIG. 2 shows the binding of two bispecific antibodies of the present disclosure and a reference antibody to stably-transfected HEK293 cells expressing hCLDN18.2, respectively.
FIG. 3 shows the binding of two bispecific antibodies of the present disclosure and a reference antibody to Jurkat cells naturally expressing hCD3, respectively.
FIG. 4 shows the binding of two bispecific antibodies of the present disclosure and a reference antibody to stably-transfected HEK293 cells expressing hCLDN18.1, respectively.
FIG. 5 shows the binding of two bispecific antibodies of the present disclosure and a reference antibody to stably-transfected HEK293 cells expressing mCLDN18.2, respectively.
FIG. 6 shows the binding of two bispecific antibodies of the present disclosure and a reference antibody to stably-transfected HEK293 cells expressing mCLDN18.1, respectively.
FIG. 7 shows the binding of two bispecific antibodies of the present disclosure and a reference antibody to stably-transfected HEK293 cells expressing cynoCLDN18, respectively.
FIG. 8 shows the results of cytotoxicity experiments (TDCC) of CD3⁺T cells against stably-transfected HEK293 cells expressing hLDN18.2 mediated by two bispecific antibodies of the present disclosure.
FIG. 9 shows the results of T cell activation of two bispecific antibodies of the present disclosure on stably-transfected HEK293 cells expressing hCLDN18.2.
FIG. 10 shows the results of INF-γ secretion by two bispecific antibodies of the present disclosure on stably-transfected HEK293 cells expressing hCLDN18.2.
FIG. 11 shows the results of IL-2 secretion by two bispecific antibodies of the present disclosure on stably-transfected HEK293 cells expressing hCLDN18.2.
FIG. 12 shows the results of IL-6 secretion by two bispecific antibodies of the present disclosure on stably-transfected HEK293 cells expressing hCLDN18.2.
FIG. 13 shows the results of TNF-α secretion by two bispecific antibodies of the present disclosure on stably-transfected HEK293 cells expressing hCLDN18.2.
FIG. 14 shows the results of luciferase expression levels of two bispecific antibodies of the present disclosure against the Jurkat-NFAT reporter gene.
FIG. 15 shows the results of cytotoxicity experiments (ADCC) of NK cells against stably-transfected HEK293 cells expressing hCLDN18.2 mediated by two bispecific antibodies of the present disclosure.
FIG. 16 shows the effect of bispecific antibody 31905-44AA of the present disclosure on tumor growth in the humanized HEK293-hCLDN18.2 model.
FIG. 17 shows the effect of bispecific antibody 31905-44AA of the present disclosure on body weight of tumor-bearing mice in the humanized HEK293-hCLDN18.2 model.

### Detailed Description of the Disclosure

All publications, patents, and patent applications mentioned in this specification are herein incorporated by reference to the same extent as if each individual publication, patent, or patent application was specifically and individually indicated to be incorporated by reference.

Before the present disclosure is described in detail below, it is to be understood that the present disclosure is not limited to the particular methodology, protocols, and reagents described herein, as these may vary. It is also to be understood that the terms used herein are for the purpose of describing particular embodiments only and are not intended to limit the scope of the present disclosure. Unless otherwise specified, all technical and scientific terms used herein have the same meanings as those generally understood by a person of ordinary skill in the art to which the present disclosure belongs.

Certain embodiments disclosed herein encompass numerical ranges, and certain aspects of the present disclosure may be described in terms of ranges. Unless otherwise indicated, it is to be understood that numerical ranges or descriptions of ranges are merely for brevity and convenience and should not be construed as strictly limiting the scope of the present disclosure. Accordingly, the description in range format should be taken to specifically disclose all possible subranges and all possible specific numerical points within that range, as such subranges and numerical points are expressly written herein. The above principles are equally applicable regardless of the width of the numerical values. Where a range description is employed, the range includes the endpoints of the range.

The term "about" when referring to a measurable value such as an amount and temporal duration, refers to a change that includes ± 20%, or in some cases ± 10%, or in some cases ± 5%, or in some cases ± 1%, or in some cases ± 0.1% of the specified value.

Amino acid three-letter codes and one-letter codes as used herein are as described in J.Biol.Chem, 243, p3558(1968).

As used herein, the terms "Claudin18.2" or "CLDN18.2" or "CLDN18A2" have the following meanings. The tight junction protein 18 (also referred to as claudin 18, abbreviated as CLDN18) molecule is an integral membrane protein (tetraspanin) with four transmembrane hydrophobic regions and two extracellular loops (loop 1 is surrounded by hydrophobic regions 1 and 2; loop 2 is surrounded by hydrophobic regions 3 and 4). CLDN18 exists as two different splice variants, which are described in mice and humans (Niimi, Mol.Cell.Biol. 21:7380-90, 2001). The GenBank accession numbers for splice variant 1 (Claudin18.1, CLDN18.1, CLDN 18A1) are NP_057453 and NM_016369; the GenBank accession numbers for splice variant 2 (Claudin18.2, CLDN18.2, CLDN 18A2) are NP_001002026 and NM_001002026. The splice variants CLDN18.1 and CLDN18.2 differ in the N-terminal portions comprising a first transmembrane (TM) region and loop 1, but have identical primary protein sequences at the C-terminus.

As used herein, the terms "anti-Claudin18.2 antibody", "anti-CLDN18A2 antibody", "anti-CLDN18.2 antibody", or "antibody against CLDN18.2" refers to an antibody that is capable of binding to a CLDN18.2 protein or fragment thereof with sufficient affinity, without significantly binding to CLDN18.1, such that the antibody can be used as a diagnostic and/or therapeutic agent that targets CLDN18.2. The human-derived CLDN18.2 protein is designated as hCLDN18.2, thus, "anti-human Claudin18.2 antibody", "anti-human CLDN18A2 antibody", "anti-hCLDN18.2 antibody", or "antibody against hCLDN18.2" in particular to refers to such an antibody that is capable of binding to the human CLDN18.2 protein or fragment thereof with sufficient affinity such that the antibody can be used as a diagnostic and/or therapeutic agent that targets human CLDN18.2.

Also, in addition to the human-derived CLDN18.2 protein, the murine-derived CLDN18.2 protein is denoted as mCLDN18.2, and the Cynomolgus macaques-derived CLDN18 protein is denoted as cynoCLDN18. Similarly, the human-derived CLDN18.1 protein is designated as hCLDN18.1 and the murine-derived CLDN18.1 protein is designated mCLDN18.1.

"CD3" is known in the art as a six-chain multiprotein complex (see Abbas and Lichtman, 2003; Janeway et al., p172 and 178, 1999). In mammals, the complex comprises a CD3 (γ) chain, a CD3 (δ) chain, and two CD3 (ε) chains and a homodimer of two CD3 (ζ) chains. The CD3 (γ), CD3 (δ), and CD3 (ε) chains are highly related cell surface proteins of the immunoglobulin superfamily comprising a single immunoglobulin domain. The transmembrane regions of the CD3 (γ), CD3 (δ), and CD3 (ε) chains are negatively charged, a feature that allows these chains to bind to positively charged T cell receptor chains. The intracellular tails of the CD3 (γ), CD3 (δ), and CD3 (ε) chains each contain a conserved motif called immunoreceptor-based tyrosine activation motif or ITAM, whereas each CD3 (ζ) chain contains three conserved motifs. Without wishing to be bound by theory, it is believed that ITAM is important for the signal transduction capacity of the TCR complex. When only the expression "CD3" is used herein, it may be derived from different animal species, including humans, mice, rats, or other mammals.

The term "anti-CD3 antibody", as used herein, refers to an antibody that specifically binds to an individual CD3 chain (e.g., a CD3 (γ) chain, a CD3 (δ) chain, or a CD3 (ε) chain) or a complex formed from two or more individual CD3 chains (e.g., a complex of more than one CD3 (ε) chain, a complex of a CD3 (γ) chain and a CD3 (ε) chain, a complex of a CD3 (δ) chain and a CD3 (ε) chain). In certain embodiments, the anti-CD3 antibody specifically binds to CD3 (γ), CD3 (δ), or CD3 (ε), or any combination thereof, more preferably, specifically binds to CD3 (ε). Human-derived CD3 is denoted as hCD3, thus, "anti-human CD3 antibody" and "anti-hCD3 antibody" refer to antibodies that specifically bind to human-derived CD3.

The term "antibody", as used herein, typically refers to a Y-type tetrameric protein comprising two heavy (H) polypeptide chains (HC) and two light (L) polypeptide chains (LC) held together by covalent disulfide bonds and non-covalent interactions. Native IgG antibodies have such a structure. Each light chain consists of one variable domain (VL) and one constant domain (CL). Each heavy chain comprises one variable domain (VH) and one constant region (CH).

As known in the art, an antibody may be classified into IgA, IgD, IgE, IgG, and IgM, respectively, with the corresponding heavy chain constant domains called α, δ, ε, γ, and µ, respectively; and IgG, and IgA may be further classified into different subclasses, IgG may be subdivided into for example IgG1, IgG2, IgG3, and IgG4, and IgA may be subdivided into IgA1 and IgA2. The light chains of antibodies from any vertebrate species can be assigned to one of two distinct types, called κ and λ, based on the amino acid sequences of their constant domain.

In IgA, IgG, and IgD, the constant region comprises three domains called CH1, CH2, and CH3 (IgM and IgE have the forth domain CH4). In IgG, IgA, and IgD, the CH1 and CH2 domains are isolated by a flexible hinge region, which is a proline and cysteine-rich segment of variable length. Each type of antibodies further comprises interchain and intrachain disulfide bonds formed by paired cysteine residues.

The term "variable region" or "variable domain" shows a significant change in amino acid composition from one antibody to another and is primarily responsible for antigen recognition and binding. The variable region of each light/heavy chain pair forms an antibody binding site such that the intact IgG antibody has two binding sites (i.e., it is bivalent). The variable region of the heavy chain (VH) and the variable region of the light chain (VL) domains each comprise three regions of extreme variability, which are termed hypervariable regions (HVRs), or more generally, complementarity-determining regions (CDRs), each VH and VL having four FRs (or framework regions), denoted FR1, FR2, FR3, and FR4, respectively. Thus, CDR and FR sequences typically occur in the following sequences of variable region of heavy chain (VH) (or variable region of light chain (VL)): FR1-HCDR1 (LCDR1)-FR2-HCDR2 (LCDR2)-FR3-HCDR3 (LCDR3)-FR4.

The term "Fc" is used herein to define the C-terminal region of an immunoglobulin heavy chain, i.e., two polypeptide chains forming a dimer comprising a C-terminal constant region capable of stabilizing self-association in an immunoglobulin heavy chain. This term includes native sequence Fc regions and variant Fc regions. Although the boundaries of the Fc region of an IgG heavy chain may vary slightly, the Fc region of a human IgG heavy chain is generally defined as extending from Cys226 or Pro230 to the carboxy terminus of the heavy chain, e.g., the IgG Fc domain comprises the IgG CH2 and IgG CH3 constant domains. Unless otherwise specified herein, numbering of amino acid residues in the Fc region or constant region follows the EU numbering system, also referred to as the EU index, as described in Kabat et al., Sequences of Proteins of Immunological Interest, 5th Ed., Public Health Service, National Institutes of Health, Bethesda, MD, 1991.

However, the antibody produced by the host cell may be subjected to post-translational cleavage to cleave one or more, particularly one or two, amino acids from the C-terminus of the heavy chain. An antibody produced by a host cell by expression of a particular nucleic acid molecule encoding a full-length heavy chain may include the full-length heavy chain, or it may include a cleavage variant of the full-length heavy chain. This may be the case when the final two C-terminal amino acids of the heavy chain are glycine (G446) and lysine (K447). Thus, the C-terminal lysine (K447), or the C-terminal glycine (G446) and lysine (K447) of the Fc region may be present or absent. To prevent C-terminal cleavage of the Fc region resulting in loss of the other antigen-binding domains fused thereto, in one embodiment of the present disclosure, K447 is substituted with amino acid A, i.e., K447A.

As used herein, the types of "antibodies" in a broad sense may include, for example, polyclonal antibodies, monoclonal antibodies, chimeric antibodies, humanized and primatized antibodies, CDR-grafted antibodies, human antibodies (including recombinantly produced human antibodies), recombinantly produced antibodies, intracellular antibodies, multispecific antibodies, bispecific antibodies, monovalent antibodies, multivalent antibodies, anti-idiotypic antibodies, synthetic antibodies (including muteins and variants thereof), etc.

The terms "full-length antibody" and "intact antibody" are used interchangeably herein to refer to an antibody having a structure substantially similar to that of a native antibody structure or having an Fc region.

The term "monoclonal antibody" (or "mAb") refers to a substantially homogeneous antibody produced by a single cell clone that is directed against only a particular antigenic epitope. Monoclonal antibodies can be prepared using a variety of techniques known in the art, including hybridoma techniques, recombinant techniques, phage display techniques, transgenic animals, synthetic techniques, or combinations thereof.

The term "chimeric antibody" is a construct in which a portion of the heavy and/or light chain is identical or homologous to a corresponding sequence in an antibody from a particular species or belonging to a particular antibody class or subclass, and the remaining portion of the chain(s) is identical or homologous to a corresponding sequence in an antibody from another species or belonging to another antibody class or subclass, and corresponding sequences in fragments of such antibodies. In a narrow sense, a chimeric antibody comprises all or most of selected murine heavy and light chain variable regions operably linked to human light and heavy chain constant regions. The constant region sequences, or variants or derivatives thereof, may be operatively associated with the disclosed heavy and light chain variable regions using standard molecular biology techniques to provide full-length antibodies that may be used themselves or may be incorporated into the anti-CLDN18.2 of the present disclosure.

The term "humanized antibody" is a hybrid immunoglobulin, immunoglobulin chain or fragment thereof that contains the smallest sequence derived from a non-human immunoglobulin. In most cases, the humanized antibody is a human immunoglobulin (recipient antibody) in which residues from CDRs of the recipient are replaced by residues from CDRs of a non-human species (donor antibody) having the desired specificity, affinity and properties, such as mice, rats, rabbits or primates. In some cases, the framework residues of a human immunoglobulin are replaced with corresponding non-human residues. In some cases, "back mutations" may be introduced into a humanized antibody in which residues in one or more FRs of the variable region of the recipient human antibody are replaced with corresponding residues from a non-human species donor antibody. Such back mutations may help maintain the proper three-dimensional configuration of one or more grafted CDRs, thus improving affinity and antibody stability. The antibody from a variety of donor species including, but not limited to, mice, rats, rabbits, or non-human primates may be used. In addition, the humanized antibody may contain new residues not found in the recipient antibody or in the donor antibody to further improve antibody performance.

It is noted that the divisions of CDR and FR in the variable regions of the antibody of the present disclosure are determined according to the Kabat definition. However, other naming and numbering systems, such as Chothia, IMGT, or AHo, are also known to those skilled in the art. Thus, the humanized antibody comprising one or more CDRs derived from any nomenclature system, based on the antibody sequences of the present disclosure, are expressly maintained within the scope of the present disclosure.

The term "sequence identity" or "sequence similarity" or "sequence homology" refers to the percentage of amino acid residues in a candidate sequence that are identical to the same amino acid residues in a reference polypeptide sequence after the sequences are aligned (and gaps are introduced when necessary) to achieve the maximum percent sequence identity, and any conservative substitutions are not considered as part of the sequence identity. Sequence alignments can be performed using various approaches in the art to determine percent amino acid sequence identity, for example, using publicly available computer software such as BLAST, BLAST-2, ALIGN, or MEGALIGN (DNASTAR) software. Those skilled in the art can determine the appropriate parameters for the measurement alignment, including any algorithm required to achieve the maximum alignment over the full-length of the sequence being compared.

The term "antibody fragment" encompasses at least a portion of an intact antibody. As used herein, a "fragment" of an antibody molecule includes an "antigen-binding fragment" of an antibody, and the term "antigen-binding fragment" refers to a polypeptide fragment of an immunoglobulin or antibody that specifically binds to or reacts with a selected antigen or antigenic epitope thereof, or a fusion protein product further derived from the fragment, e.g., a single chain antibody, an extracellular binding region in a chimeric antigen receptor, etc. Exemplary antibody fragments or antigen-binding fragments thereof include, but are not limited to: light chain variable fragments (VL), heavy chain variable fragments (VH), Fab fragments, F(ab')₂ fragments, Fd fragments, Fv fragments, single domain antibodies, linear antibodies, single chain antibodies (scFv), bispecific antibodies, or multispecific antibodies formed from antibody fragments, etc.

The term "antigen-binding domain" or "binding domain" refers to a domain that specifically binds to/interacts with/recognizes a given target epitope on a target molecule (antigen). An "antigen-binding domain" can be either a single "antigen-binding fragment" or a combination of "antigen-binding fragments", which is a broader concept than "antigen-binding fragments".

The term "Fab fragment" includes a variable region of each of the heavy and the light chain, and also includes a constant region of the light chain and a first constant region CH1 of the heavy chain, which is a monovalent antibody fragment. The term "F(ab')₂ fragment" encompasses two Fab fragments as well as hinge regions, which is a bivalent antibody fragment.

The term "Fd fragment" generally encompasses a heavy chain variable region and a constant region CH1; the term "Fv fragment" is the smallest antibody fragment having variable regions of heavy chain and light chain, but no constant region, and holding a complete antigen-binding sites.

The term "single domain antibody", also known as Nanobody, is a naturally occurring antibody devoid of light chains in the peripheral blood of alpaca, comprising only one heavy chain variable VHH and two conventional CH2 and CH3 regions. The VHH structure cloned and expressed separately, which has structural stability equivalent to that of the original heavy chain antibody and binding activity to an antigen, is known as the smallest unit binding to a target antigen, and is therefore called Nanobody (Nb).

The term "scFv" refers to a fusion protein comprising at least one antibody fragment including the variable region of the light chain and at least one antibody fragment including the variable region of the heavy chain, wherein the variable regions of the light and heavy chain are connected (e.g., via a synthetic linker such as a short flexible polypeptide linker) and capable of being expressed as a single chain polypeptide, and wherein the scFv retains the specificity of the intact antibody from which it is derived. Unless otherwise specified, an scFv may have the VL and VH variable regions described in any order (e.g., relative to the N-terminus and C-terminus of the polypeptide), and an scFv may comprise a VL-linker-VH or may comprise a VH-linker-VL.

Naturally occurring antibodies are generally monospecific, i.e., they bind to a single antigen. The present disclosure provides molecules that bind to both cytotoxic cells (e.g., CD3 on T cells) and target cells (e.g., CLDN18.2 on cancer cells) simultaneously. The molecule binds to at least two different types of antigens and is at least bispecific or multispecific. The binding molecules of the present disclosure may be at least trivalent. As used herein, "valent" means the number of antigen binding sites in a molecule, e.g., a typical native IgG antibody is bivalent. The antigen binding sites that bind to the same antigen may recognize the same epitope or different epitopes. Trivalent bispecific antibodies and tetravalent bispecific antibodies are known in the art.

The term "multispecific antibody" refers to a novel antibody construct binding to more than two different sites and/or targets, which is formed by functionally linking (e.g., chemical coupling, gene fusion, non-covalent binding, or other methods) the antibody or antibody fragment to one or more other binding molecules (including antibodies or antibody fragments or other molecules with binding capacity). Thus, a "bispecific antibody" (alternatively referred to as a "bispecific antigen binding molecule" or "diabody") specifically refers to an antibody construct having specificity for two different antigens and/or epitopes. Typically, a bispecific or multispecific antibody includes at least two different antigen (or epitope) binding domains.

In the context of the present disclosure, the term "recombinant" means prepared by genetic engineering". In general, the recombinant is not naturally occurring.

Techniques for generating multispecific antibodies include, but are not limited to, recombinant co-expression of two immunoglobulin heavy-light chain pairs having different specificities (see Milstein and Cuello,Nature 305:537(1983)) ,WO93/08829, and Traunecker et al., EMBO J. 10:3655(1991)), and "knob-into-hole" engineering (see, e.g., U.S. Patent No. 5, 731, 168). Multispecific antibodies can also be generated by: engineered electrostatic manipulation effects for the generation of antibody Fc-heterodimer molecules (WO2009/089004A1); crosslinking two or more antibodies or fragments (see, e.g., U.S. Pat. No. 4,676,980, and Brennan et al., Science, 229:81(1985)); using the leucine zipper to generate bispecific antibodies (see, e.g., Kostelny et al., J .Immunol., 148(5):1547-1553(1992)); using the "diabody" technique for the generation of bispecific antibody fragments (see, e.g., Hollinger et al., Proc. Natl. Acad. Sci. USA, 90:6444-6448 (1993)); using single chain Fv (sFv) dimers (see, e.g., Gruber et al., J. Immunol., 152:5368, 1994)); and, for example, by preparing trispecific antibodies as described in Tutt et al., J. Immunol. 147:60(1991).

The bispecific antibodies of the present disclosure may have either full-length antibodies or intact antibodies as scaffolds to which various scFv are added, e.g., scFv is fused at the C-terminus of the heavy chain CH3 domain or the C-terminus of the light chain CL domain.

The term "fused" or "linked" means that the components (e.g., antigen-binding fragment or antigen-binding domain) are linked together by a peptide bond, either directly or via one or more peptide linkers.

As used herein, "homologous" and "heterologous" are a set of relative concepts, which may refer to different elements in a construct having the same or different sources, or to multiple elements that are originally derived from the same source, i.e., "homologous", after the construct has been constructed, some elements having been modified and changed from other original elements that have not been modified, thereby becoming "heterologous".

In one embodiment, when scFv is fused to the C-terminus of the light chain CL domain of a full-length antibody or an intact antibody, then a bispecific antibody of the present disclosure comprises four polypeptide chains, two homologous light chains and two homologous heavy chains, respectively. Wherein, a homologous light chain polypeptide comprises VL-CL-scFv domains from N-terminus to C-terminus, and a homologous heavy chain polypeptide comprises VH-CH domains from N-terminus to C-terminus.

In one embodiment, when scFv is fused to the C-terminus of the one heavy chain CH3 domain of a full-length antibody or an intact antibody, then a bispecific antibody of the present disclosure comprises four polypeptide chains, two homologous light chains and two heterogenous heavy chains, respectively. Wherein the homologous light chain polypeptide comprises VL-CL domains from N-terminus to C-terminus, one of the heterologous heavy chain polypeptides comprises VH-CH domains from N-terminus to C-terminus, and the other of the heterologous heavy chain polypeptides comprises VH-CH-scFv domains from N-terminus to C-terminus.

The term "linker" refers to any means for linking two different functional units (e.g., antigen-binding fragments). Types of linkers include, but are not limited to, chemical linkers and polypeptide linkers. The sequence of the polypeptide linker is not limited. The polypeptide linker is preferably non-immunogenic and flexible, such as those comprising serine and glycine sequences. Depending on the particular construct, the linker may be long or short.

According to the present disclosure, the linker linking the different functional units preferably comprises a flexible peptide linker, such as a glycine-serine peptide linker. In one embodiment, the linker comprises the amino acid sequence (G4S)x, wherein x is selected from any integer from 1 to 6, preferably comprises the amino acid sequence (G4S)i or (G4S)₃. The linker linking the VH and VL domains to form the scFv domains of either VH-VL or VL-VH preferably comprises a flexible peptide linker, for example, a glycine-serine peptide linker. In one embodiment, the linker comprises the amino acid sequence (G4S)x, wherein x is selected from any integer from 1 to 6, preferably comprises the amino acid sequence (G4S)₃.

The term "antigen" refers to a substance recognized and specifically bound by an antibody or antibody-binding fragment, and broadly, an antigen can include any immunogenic fragment or determinant of a selected target, including a single epitope, a multi-epitope, a single domain, a multiple domain, or an entire extracellular domain (ECD) or a protein. Peptides, proteins, glycoproteins, polysaccharides and lipids, portions thereof and combinations thereof may constitute antigens. Non-limiting exemplary antigens include tumor antigens or pathogen antigens, etc. "Antigen" may also refer to a molecule that triggers an immune response. Any form of antigens or cells or preparations containing the antigens may be used to generate antibodies specific for an antigenic determinant. The antigen can be an isolated full-length protein, a cell surface protein (e.g., immunized with a cell expressing at least a portion of the antigen on its surface), or a soluble protein (e.g., immunized with only the ECD portion of the protein), or a protein construct (e.g., an Fc antigen). The antigen may be produced in genetically modified cells. Any of the foregoing antigens may be used alone or in combination with one or more immunogenicity-enhancing adjuvants known in the art. The DNA encoding the antigen may be genomic or non-genomic (e.g., cDNA) and may encode at least a portion of the ECD sufficient to trigger an immunogenic response. Any vector may be used to transform cells in which the antigen is expressed, including but not limited to adenoviral vectors, lentiviral vectors, plasmids, and non-viral vectors such as cationic lipids.

The term "epitope" refers to a site on an antigen that specifically binds to an immunoglobulin or antibody. Epitopes may be formed from contiguous amino acids or noncontiguous amino acids juxtaposed by tertiary folding of a protein. Epitopes formed from contiguous amino acids are typically retained upon exposure to denaturing solvents, while epitopes formed by tertiary folding are typically lost upon treatment with denaturing solvents. Epitopes typically exist in a unique spatial conformation and comprise at least 3-15 amino acids. Methods for determining the epitope to which a given antibody binds are well known in the art, including immunoblotting and immunoprecipitation detection assays. Methods for determining the spatial conformation of an epitope include techniques in the art and described herein, such as X-ray crystallography, two-dimensional nuclear magnetic resonance, etc.

The terms "polypeptide", "peptide", and "protein" are used interchangeably herein to refer to polymers of amino acids of any length. The polymer may be linear, cyclic or branched, and may comprise modified amino acids, particularly conservatively modified amino acids, and it may be interrupted by non-amino acids. The term also includes modified amino acid polymers such as amino acid polymers that have been modified by sulfation, glycosylation, lipidation, acetylation, phosphorylation, iodination, methylation, oxidation, proteolytic processing, prenylation, racemization, selenoylation, transfer RNA (tRNA)-mediated amino addition such as arginate, ubiquitination, or any other operation such as conjugation to a labeling component. As used herein, the term "amino acid" refers to natural and/or non-natural or synthetic amino acids, including glycine and D or L optical isomers, as well as amino acid analogs and peptidomimetics. A polypeptide or amino acid sequence "derived from" a given protein refers to the source of the polypeptide. The term also includes polypeptides expressed from the specified nucleic acid sequences.

The term "amino acid modification" (or "modified amino acid") includes amino acid substitutions, insertions, and/or deletions in a polypeptide sequence. As used herein, "amino acid substitution" or "substitution" or "substituting" refers to the replacement of an amino acid at a particular position in a parent polypeptide sequence with another amino acid. For example, substitution S32A means that serine at position 32 is replaced with alanine.

Sequence identity or homology of a humanized antibody variable region to a human receptor variable region can be determined as discussed herein, and when measured in this way, the two will preferably share at least 60% or 65% sequence identity, more preferably at least 70%, 75%, 80%, 85% or 90% sequence identity, even more preferably at least 93%, 95%, 98% or 99% sequence identity. Preferably, residue positions that are not identical differ by conservative amino acid substitutions. A "conservative substitution" is an amino acid substitution in which one amino acid residue is replaced with another amino acid residue having a side chain (R group) with similar chemical properties (e.g., charge or hydrophobicity). In general, conservative amino acid substitutions do not substantially alter the functional properties of the protein. Families of amino acid residues having similar side chains have been defined in the art. These families include amino acids containing basic side chains (e.g., lysine, arginine, histidine), acidic side chains (e.g., aspartic acid, glutamic acid), uncharged polar side chains (e.g., glycine, asparagine, serine, threonine, tyrosine, cysteine, tryptophan), non-polar side chains (e.g., alanine, valine, leucine, isoleucine, proline, phenylalanine, methionine), β branched side chains (e.g., threonine, valine, isoleucine), and aromatic side chains (e.g., tyrosine, phenylalanine, tryptophan, histidine). Thus, one or more amino acid residues in the CDR regions or in the framework regions of the antibodies of the present disclosure may be replaced with amino acid residues of other similar side chains. In the case where two or more amino acid sequences differ from one another by conservative substitutions, the percent sequence identity or degree of similarity may be adjusted upward to correct for the conservative nature of the substitution.

In the production of antibodies, various post-translational modifications (PTM) variants, such as glycosylation, oxidation, saccharification, deamidation, isomerization and end-group cyclization, are easily produced by different physical and chemical factors.

These PTMs may cause changes in the physical and chemical properties of the antibody, alter the interaction with the Fc receptor of the antibody, and affect the binding activity with the target antigen; the occurrence of some PTMs may even reduce antibody stability, cause immunogenicity, etc. (JARASCH et al., JOURNAL OF PHARMACEUTICAL SCIENCES, 2015). Negative effects can be eliminated by amino acid modifications, such as conservative substitutions, to the PTM site. Amino acid substitutions to antibody CDRs to modify PTM are also clearly remain the scope of the present disclosure.

The term "antibody-dependent cell-mediated cytotoxicity" (ADCC) refers to the binding of an antibody to an epitope of a virus-infected cell or tumor cell, wherein Fc fragment binds to Fc receptors (FcRs) present on certain killer cells (e.g. NK cells, and macrophages, etc.) to mediate the killer cells to directly kill target cells.

The term "complement dependent cytotoxicity" (CDC) refers to the cytotoxic effect in the presence of complement, i.e., the lysis of target cells by membrane attack complex formed by activation of the classical complement pathway, which is initiated by the binding of specific antibodies to corresponding membrane surface antigens.

Bispecific antibodies of the present disclosure may also include substitutions or modifications of constant regions (e.g., Fc), including, but not limited to, amino acid residue substitutions, mutations, and/or modifications, which result in compounds having the following preferred characteristics, including, but not limited to: altered pharmacokinetics, increased serum half-life, increased binding affinity, decreased immunogenicity, increased yield, altered Fc ligand binding to Fc receptors (FcRs), increased or decreased ADCC or CDC, altered glycosylation and/or disulfide bonds, and modified binding specificity. In certain aspects, an antibody variant comprises an Fc region having one or more amino acid substitutions that impair FcyR binding (e.g., substitutions at positions 234 and 235 of the Fc region). In one aspect, the substitutions are L234A and L235A.

A bispecific antibody according to the present disclosure comprises a scFv fragment fused to one or the other of the C-termini of the Fc domain of a full-length antibody, such that two heterologous Fc domain-containing polypeptide chains are formed. Recombinant co-expression and subsequent dimerization of these polypeptides results in several possible combinations of the two polypeptides. To improve the yield and purity of bispecific antibodies in recombinant production, it may be advantageous to introduce modifications in the Fc domain of the bispecific antibodies that promote the association of desired polypeptides. Thus, in a particular embodiment, the Fc domain of the bispecific antibodies according to the present disclosure comprises a modification that promote the association of the first polypeptide chain and the second polypeptide chain of the Fc domain. The site of the most extensive protein-protein interaction between the two polypeptide chains of the human IgG Fc domain is in the CH3 domain of the Fc domain. Thus, in one embodiment, the modification is in the CH3 domain of the Fc domain.

There are several ways to modify the CH3 domain of the Fc domain to enhance heterodimerization, which are described in detail in e.g., WO96/27011, WO98/050431, EP1870459, WO2007/110205, WO2007/147901, WO2009/089004, WO2010/129304, WO2011/90754, WO2011/143545, WO2012/058768, WO2013/157954, and WO2013/096291. Typically, in all such approaches, both the CH3 domain of the first polypeptide chain of the Fc domain and the CH3 domain of the second polypeptide chain of the Fc domain are engineered in a complementary manner such that each CH3 domain (or the heavy chain comprising CH3 domain) is no longer homodimerize with itself but is forced to heterodimerize with other complementarily engineered CH3 domains (such that the first and second CH3 domains are heterodimerized and no homodimer is formed between the two first CH3 domains or the two second CH3 domains).

In a particular embodiment, the modification facilitating the association of the first polypeptide chain and the second polypeptide chain of the Fc domain is a so-called "knob-into-hole" modification, comprising a "knob" modification in one of the two polypeptide chains of the Fc domain and a "hole" modification in the other of the two polypeptide chains of the Fc domain.

Knob-into-hole techniques are described, for example, in US 5,731,168; US 7,695,936; Ridgway et al., Prot Eng 9, 617-621(1996) and Carter, J Immunol Meth 248,7-15(2001). Generally, the method involves introducing a protuberance ("knob") at the interface of a first polypeptide chain and a corresponding cavity ("hole") at the interface of a second polypeptide chain, such that the protuberance can be placed in the cavity to promote heterodimer formation and block homodimer formation. The protuberance is constructed by replacing a small amino acid side chain from the first polypeptide chain interface with a larger side chain, such as tyrosine or tryptophan. A complementary cavity having the same or similar size as the protuberance is created at the interface of the second polypeptide chain by replacing the large amino acid side chain with a smaller amino acid side chain, such as alanine or threonine.

Thus, in a particular embodiment, in the CH3 domain of the first polypeptide chain of the Fc domain of the bispecific antibody of the present disclosure, one amino acid residue is replaced with an amino acid residue having a larger side chain volume, thereby generating a protuberance in the CH3 domain of the first polypeptide chain, which can be placed in a cavity in the CH3 domain of the second polypeptide chain, and in the CH3 domain of the second polypeptide chain of the Fc domain, one amino acid residue is replaced with an amino acid residue having a smaller side chain volume, thereby generating a cavity in the CH3 domain of the second polypeptide chain, in which the protuberance in the CH3 domain of the first polypeptide chain can be placed.

Preferably, said amino acid residue having a larger side chain volume is selected from the following group: arginine (R), phenylalanine (F), tyrosine (Y), and tryptophan (W). Preferably, said amino acid residue having a smaller side chain volume is selected from the following group: alanine (A), serine (S), threonine (T), and valine (V).

Protuberances and cavities can be generated by altering the nucleic acid encoding the polypeptide, for example, by site-specific mutagenesis or by peptide synthesis.

In a particular embodiment, the threonine residue at position 366 is replaced with a tryptophan residue (T366W) in the CH3 domain of the first polypeptide chain of the Fc domain (the "knob" polypeptide chain), and the tyrosine residue at position 407 is replaced with a valine residue (Y407V) in the CH3 domain of the second polypeptide chain of the Fc domain (the "hole" polypeptide chain). In one embodiment, in the second polypeptide chain of the Fc domain, in addition, the threonine residue at position 366 is replaced with a serine residue (T366S) and the leucine residue at position 368 is replaced with an alanine residue (L368A).

In another embodiment, in the first polypeptide chain of the Fc domain, in addition, the serine residue at position 354 is replaced with a cysteine residue (S354C) or the glutamate residue at position 356 is replaced with a cysteine residue (E356C), and in the second polypeptide chain of the Fc domain, in addition, the tyrosine residue at position 349 is replaced with a cysteine residue (Y349C). The introduction of these two cysteine residues results in the formation of a disulfide bridge between the two polypeptide chains of the Fc domain, further stabilizing the dimer (Carter,J Immunol Methods 248,7-15(2001)).

In a particular embodiment, the first polypeptide chain of the Fc domain comprises the amino acid substitutions S354C and T366W, and the second polypeptide chain of the Fc domain comprises the amino acid substitutions Y349C, T366S, L368A, and Y407V.

To facilitate purification of polypeptide chain homodimers with "holes" from the final Fc domain-containing bispecific heterodimers molecules, other substitutions that may be made to the CH3 domain include those that affect binding to protein A. Non-limiting examples of such substitutions include H435R, H435R, and/or Y436F (U.S. Patent No: US 8,586,713 B2). The protein A binding domain of CH2 and CH3 domains of the polypeptide chain with a "hole" is preferably mutated by an amino acid substitution at position 435 (H435R). Thus, a polypeptide chain homodimer with a "hole" will not bind to protein A, while a bispecific heterodimer will retain its ability to bind to protein A via a protein A binding domain.

The term "specificity" means that an antibody is selective for binding to an antigen and can be distinguished from unwanted or non-specific interactions.

The term "affinity" or "binding affinity" refers to the strength of the sum of all non-covalent interactions between a single binding site of a molecule (e.g., an antibody) and its binding partner (e.g., an antigen). The term "K_{D}" refers to the dissociation constant of a particular antibody-antigen interaction. Binding affinities can be determined using various techniques known in the art, such as surface plasmon resonance, bio-layer interferometry, dual polarization interferometry, static light scattering, dynamic light scattering, isothermal titration calorimetry, ELISA, analytical ultracentrifugation, and flow cytometry, etc.

The term "pharmaceutical composition" refers to a formulation that is present in a form that allows the biological activity of the active ingredients contained therein to be effective, and which does not contain additional ingredients having unacceptable toxicity to the subject to which the formulation is administered.

The term "pharmaceutically carrier" or "pharmaceutically acceptable carrier" refers to a diluent, adjuvant (e.g., Freund's adjuvant (complete and incomplete)), excipient, or vehicle with which a therapeutic agent is administered.

The term "effective amount" refers to a dose of a pharmaceutical formulation of active ingredients of the present disclosure that, when administered to a patient in a single or multiple doses, produces the desired effect in the treated patient. An effective amount can be readily determined by the attending physician, as one skilled in the art, by considering the following factors: such as the different of human species; body weight, age and health; specific diseases involved; the severity of the disease; response of an individual patient; the specific antibody administered; modes of administration; bioavailability characteristics of the administered formulation; a selected dosing regimen; and the use of any concomitant therapy.

The terms "host cell", "host cell line" and "host cell culture" are used interchangeably and refer to a cell into which an exogenous nucleic acid is introduced, including progeny of such a cell. Host cells include "transformants" and "transformed cells", which include primarily transformed cells and progeny derived therefrom, regardless of the number of passages. The progeny may not be exactly the same as the parent cell in nucleic acid content, but may contain mutations. Mutant progeny having the same function or biological activity as screened or selected in the initially transformed cell are included herein.

As used herein, the term "transfection" refers to the introduction of an exogenous nucleic acid into a eukaryotic cell. Transfection can be accomplished by various means known in the art, including calcium phosphate-DNA co-precipitation, DEAE-dextran- mediated transfection, polybrene-mediated transfection, electroporation, microinjection, liposome fusion, lipid transfection, protoplast fusion, retroviral infection, and biolistics.

The term "stable transfection" or "ST" refers to the introduction and integration of an exogenous nucleic acid, DNA or RNA into the genome of a transfected cell. The term "stable transfectant" refers to a cell that stably integrates foreign DNA into genomic DNA.

The terms "nucleic acid molecule encoding", "coding DNA sequence" and "coding DNA" refer to the order of deoxyribonucleotides along a strand of deoxyribonucleic acid. The order of these deoxyribonucleotides determines the order of the amino acids along the polypeptide (protein) chain. Thus, the nucleic acid sequence encodes an amino acid sequence.

Methods for producing and purifying antibodies and antigen-binding fragments are well known in the art and can be found, for example, in chapters 5-8 and 15 in Using Antibodies: A Laboratory Manual, Cold Spring Harbor Laboratory. The antibodies or antigen-binding fragments thereof of the present disclosure are genetically engineered to add one or more human FR regions to CDR regions of non-human origin. Human FR germline sequences can be obtained from the website http://imgt.cines.fr of ImMunoGeneTics (IMGT), or from J. Immunoglobulin, (2001) ISBN: 012441351.

The engineered antibodies or antigen-binding fragments thereof of the present disclosure can be prepared and purified by conventional methods. For example, cDNA sequences encoding heavy and light chains can be cloned and recombined into expression vectors. The recombinant immunoglobulin expression vector can stably transfect CHO cells. As a more recommended prior art, mammalian expression systems may result in glycosylation of antibodies, particularly at the highly conserved N-terminus of the Fc region. Stable clones are obtained by expressing antibodies that specifically bind to human antigens. Positive clones are enlarged cultured in serum-free medium in a bioreactor to produce antibodies. The antibody-secreting medium may be purified and collected using conventional techniques. The antibody may be concentrated by filtration using conventional methods. Soluble mixtures and polymers may also be removed by conventional methods, such as molecular sieves, ion exchange, etc.

As used herein, the term "individual" or "subject" refers to any animal, such as a mammal or a bagged animal. Individuals of the present disclosure include, but are not limited to, humans, non-human primates (e.g., Cynomolgus macaques or Rhesus Macacus or other types of macaque), mice, pigs, horses, donkeys, cattle, sheep, rats, and any kind of poultry. As used herein, the term "tumor" refers to a disease characterized by pathological proliferation of cells or tissues, and subsequent migration or invasion of other tissues or organs. The growth of a tumor is usually uncontrolled and progressive, and does not induce or inhibit normal cell proliferation. Tumors can affect various cells, tissues or organs, including, but not limited to, bladder, bone, brain, breast, cartilage, glial cells, esophagus, fallopian tube, gallbladder, heart, intestine, kidney, liver, lung, lymph nodes, nerve tissue, ovary, pancreas, prostate, skeletal muscle, skin, spinal cord, spleen, stomach, testis, thymus, thyroid, trachea, urethra, ureter, urethra, uterus, vaginal organ, or tissue or corresponding cell. Tumors include cancers, such as sarcomas, carcinomas, or plasmacytomas (malignant tumors of plasma cells). The tumor according to the present disclosure may include, but is not limited to, leukemia (e.g. acute leukemia, acute lymphocytic leukemia, acute myeloid leukemia, acute myeloid leukemia, acute promyelocytic leukemia, acute myelo-monocytic leukemia, acute monocytic leukemia, chronic leukemia, chronic myelogenous leukemia, chronic lymphocytic leukemia, polycythemia vera), lymphoma (Hodgkin's disease, non-Hodgkin's disease), primary macroglobulinemia, heavy chain disease, solid tumors such as sarcomas and cancers (e.g. fibrosarcoma, myxosarcoma, liposarcoma, chondrosarcoma, osteosarcoma, chordoma, endothelial sarcoma, lymphangiosarcoma, angiosarcoma, lymphangioendothelioma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat adenocarcinoma, sebaceous adenocarcinoma, papillary carcinoma, papillary adenocarcinoma,bronchial carcinoma, myeloid cancer, renal cell carcinoma, liver cancer, nile duct cancer, choriocarcinoma, seminoma, embryo cancer, nephroblastoma, cervical cancer, uterine cancer, testicular cancer, lung cancer, small cell lung cancer, bladder cancer, epithelial cancer, glioma, astrocytoma, medulloblastoma, craniopharyngioma, ependymoma, pinealoma, hemangioblastoma, acoustic neuromas, oligodendroglioma, schwannoma, meningioma, melanoma, neuroblastoma, retinoblastoma), esophageal cancer, gallbladder cancer, kidney cancer, multiple myeloma. Preferably, the "tumor" includes, but is not limited to pancreatic cancer, liver cancer, lung cancer, gastric cancer, esophageal cancer, head and neck squamous cell carcinoma, prostate cancer, colon cancer, rectal cancer, breast cancer, lymphoma, gallbladder cancer, renal cancer, leukemia, multiple myeloma, ovarian cancer, cervical cancer, and glioma.

As used herein, the term "disease" or "condition" or "disorder" or the like refers to any alteration or disorder that impairs or interferes with the normal function of a cell, tissue or organ. For example, the "disease" includes, but is not limited to tumors, pathogen infections, autoimmune diseases, T-cell dysfunctions, or deficiencies in immune tolerance (e.g., transplant rejection).

As used herein, the term "treatment" refers to clinical intervention in an attempt to alter a disease caused by an individual or treated cells, either prophylactically or clinically pathologically. Therapeutic effects include, but are not limited to, prevention of the occurrence or recurrence of a disease, alleviation of symptoms, reduction of any disease's direct or indirect pathological consequences, prevention of metastasis, slowing of the rate of disease progression, amelioration or remission of a condition, remission or amelioration of a prognosis, etc.

The term "drug box" or "kit" includes an effective amount of one or more unit dosage forms of a pharmaceutical composition of the present disclosure. In some embodiments, the drug box may include a sterile container; such containers may be in the form of boxes, ampoules, bottles, vials, tubes, bags, blister packs, or other suitable containers known in the art. Such containers may be made of plastic, glass, laminated paper, metal foil or other materials suitable for holding drugs. In addition, the drug box also includes instructions for administering the pharmaceutical composition of the present disclosure to an individual. The instructions generally include methods of using the pharmaceutical compositions of the present disclosure to treat diseases.

### Detailed Description of the Invention

The present disclosure will be described in detail below in connection with specific examples. It should be understood that these examples are only used to describe the present disclosure and are not intended to limit the scope of the present disclosure. The experimental methods in the following examples which are not specified with specific conditions are generally carried out according to conventional conditions, Molecular Cloning: A Laboratory Manual (Third Edition) by J. Sambrook et al., Science Press, 2002, or according to the conditions recommended by the manufacturer.

### Example 1 Construction and expression of anti-CD3-CLDN18.2 bispecific antibodies

The bispecific antibody of the present disclosure has a structure in which the C-terminus of the heavy chain or the C-terminus of the light chain of the full-length anti-human CLDN18.2 antibody was linked to the binding domain of human T-cell receptor subunit CD3ε. Wherein, the anti-human CLDN18.2 full-length antibody was 6#AA, with the heavy chain sequence as set forth in SEQ ID NO: 1 and the light chain sequence as set forth in SEQ ID NO: 5. The CD3ε binding domain was derived from the full-length antibody h160C9AA, with the heavy chain sequences set forth in SEQ ID NO: 9 and the light chain sequence s set forth in SEQ ID NO: 10. To reduce the ADCC activity of the antibodies, the Fc fragments of both 6# AA and h160C9AA had been subjected to amino acids substitutions of L234A and L235A.

The light chain variable region and the heavy chain variable region of h160C9AA were linked via a flexible linker to form a single chain antibody scFv, with a structure of VL-(G₄S)₃-VH and a sequence as set forth in SEQ ID NO: 8. The scFv was then fused to the C-terminus of one or both of the light chains of CLDN18.2 full-length antibody 6#AA via (G4S)₃.

When scFv was fused to the C-terminus of the two light chains of 6# AA, the resulting bispecific antibody designated 31905-38AA comprises two homologous light chains and two homologous heavy chains, wherein the fused light chain has a sequence as set forth in SEQ ID NO: 2 and the heavy chain has a sequence as set forth in SEQ ID NO: 1.

When scFv was fused to the C-terminus of one heavy chain of 6#AA, the resulting bispecific antibody designated 31905-44AA comprises two homologous light chains that are and two heterologous heavy chains. Of the two heterologous heavy chains, the scFv-containing heavy chain was designed as a "knob" structure, including amino acid substitutions at two sites, S354C and T366W. Also, in order to prevent the cleavage of scFv from the C-terminus of the heavy chain, the K at the last position of the C-terminus of the heavy chain was mutated to A to perform an amino acid substitution of K447A. Of the two heterologous heavy chains, the heavy chain without scFv was designed as a "hole" structure, including four amino acid substitutions at sites Y349C, T366S, L368A, and Y407V. Also, to facilitate purification of bispecific antibodies, the heavy chain with a "hole" structure was further subjected to H435R substitution. The constructed homologous light chain has a sequence as set forth in SEQ ID NO: 5, the heavy chain with a "knob" structure has a sequence as set forth in SEQ ID NO: 3, and the heavy chain with a "hole" structure has a sequence as set forth in SEQ ID NO: 4.

The structures of bispecific antibodies of the present disclosure and the related molecular sequences were summarized in Tables 1 and 2, respectively.

**Table 1 Structures of bispecific antibodies**

| Molecular Name | Description of molecular structure | SEQ ID NO: |
|---|---|---|
| 31905-3 8AA | CLDN18.2 HC (containing IgG1 Fc) | 1 |
| | CLDN18.2 LC-(G₄S) -CD3VL-(G₄S)₃-CD3VH | 2 |
| 31905-44AA | CLDN18.2 HC (containing IgG1 Fc, knob)-(G₄S)₃-CD3VL-(G₄S)₃-CD3VH | 3 |
| | CLDN18.2 HC (containing IgG1 Fc, hole) | 4 |
| | CLDN18.2 LC | 5 |
| 6#AA | CLDN18.2 HC | 1 |
| | CLDN18.2 LC | 5 |
| h 160C9AA scFv | CD3VL-(G₄S)₃-CD3VH | 8 |
| h 160C9AA | CD3 HC | 9 |
| | CD3 LC | 10 |

**Table 2 Amino acid sequences of bispecific antibodies**

| Name | SEQ ID NO: | Amino acid sequence |
|---|---|---|
| 31905-3 8AA Heavy Chain | 1 | QVQLVQSGAEVKKPGASVKVSCKASGYIFTNYWIHWV RQAPGQGLEWMGRIYPGTGNTYYNEKFTGRVTMTRDT STSTVYMELSSLRSEDTAVYYCAREGYGKGNSMDYWG |
| | | QGTTVTVSSASTKGPSVFPLAPSSKSTSGGTAALGCLVK DYFPEPVTVSWNSGALTSGVHTFPAVLQSSGLYSLSSVVT VPSSSLGTQTYICNVNHKPSNTKVDKKVEPKSCDKTHTC PPCPAPEAAGGPSVFLFPPKPKDTLMISRTPEVTCVVVDV SHEDPEVKFNWYVDGVEVHNAKTKPREEQYNSTYRVV SVLTVLHQDWLNGKEYKCKVSNKALPAPIEKTISKAKG QPREPQVYTLPPSRDELTKNQVSLTCLVKGFYPSDIAVEW ESNGQPENNYKTTPPVLDSDGSFFLYSKLTVDKSRWQQG NVFSCSVMHEALHNHYTQKSLSLSPGK |
| 31905-38AA Light Chain | 2 | DIVMTQSPDSLAVSLGERVTMNCKSSQSLLNAGNQKNY LTWYQQKPGQPPKLLFYWASTRESGVPDRFSGSGSGTDF TLTISSVQAEDVAVYHCQNAYYYPYTFGGGTKLEIKRTV AAPSVFIFPPSDEQLKSGTASVVCLLNNFYPREAKVQWK VDNALQSGNSQESVTEQDSKDSTYSLSSTLTLSKADYEK HKVYACEVTHQGLSSPVTKSFNRGECGGGGSAQAVVTQ EPSLTVSPGGTVTLTCRSSTGAVTTSNYANWVQQKPGQA PRGLIGGTNKRAPGTPARFSGSLLGGKAALTLSGAQPED EAEYYCALWYSNLWVFGGGTKLTVLGGGGSGGGGSGG GGSEVKLVESGGGLVQPGGSLKLSCAASGFTFNTYAMN WVRQAPGKGLEWVARIRSKYNNYATYYADSVKDRFTIS RDDAKNTLYLQMNNLRTEDTAVYYCVRHGNFGNSYVS WFAYWGQGTLVTVSS |
| Heavy chain with the "Knob" structure of 31905-44AA | 3 | QVQLVQSGAEVKKPGASVKVSCKASGYIFTNYWIHWV RQAPGQGLEWMGRIYPGTGNTYYNEKFTGRVTMTRDT STSTVYMELSSLRSEDTAVYYCAREGYGKGNSMDYWG QGTTVTVSSASTKGPSVFPLAPSSKSTSGGTAALGCLVK DYFPEPVTVSWNSGALTSGVHTFPAVLQSSGLYSLSSVVT VPSSSLGTQTYICNVNHKPSNTKVDKKVEPKSCDKTHTC PPCPAPEAAGGPSVFLFPPKPKDTLMISRTPEVTCVVVDV |
| | | SHEDPEVKFNWYVDGVEVHNAKTKPREEQYNSTYRVV SVLTVLHQDWLNGKEYKCKVSNKALPAPIEKTISKAKG QPREPQVYTLPPCRDELTKNQVSLWCLVKGFYPSDIAVE WESNGQPENNYKTTPPVLDSDGSFFLYSKLTVDKSRWQ QGNVFSCSVMHEALHNHYTQKSLSLSPGAGGGGSGGG GSGGGGSAQAVVTQEPSLTVSPGGTVTLTCRSSTGAVTT SNYANWVQQKPGQAPRGLIGGTNKRAPGTPARFSGSLL GGKAALTLSGAQPEDEAEYYCALWYSNLWVFGGGTKL TVLGGGGSGGGGSGGGGSEVKLVESGGGLVQPGGSLKL SCAASGFTFNTYAMNWVRQAPGKGLEWVARIRSKYNN YATYYADSVKDRFTISRDDAKNTLYLQMNNLRTEDTAV YYCVRHGNFGNSYVSWFAYWGQGTLVTVSS |
| Heavy chain with the "hole" structure of 31905-44AA | 4 | QVQLVQSGAEVKKPGASVKVSCKASGYIFTNYWIHWV RQAPGQGLEWMGRIYPGTGNTYYNEKFTGRVTMTRDT STSTVYMELSSLRSEDTAVYYCAREGYGKGNSMDYWG QGTTVTVSSASTKGPSVFPLAPSSKSTSGGTAALGCLVK DYFPEPVTVSWNSGALTSGVHTFPAVLQSSGLYSLSSVVT VPSSSLGTQTYICNVNHKPSNTKVDKKVEPKSCDKTHTC PPCPAPEAAGGPSVFLFPPKPKDTLMISRTPEVTCVVVDV SHEDPEVKFNWYVDGVEVHNAKTKPREEQYNSTYRVV SVLTVLHQDWLNGKEYKCKVSNKALPAPIEKTISKAKG QPREPQVCTLPPSRDELTKNQVSLSCAVKGFYPSDIAVE WESNGQPENNYKTTPPVLDSDGSFFLVSKLTVDKSRWQ QGNVFSCSVMHEALHNRYTQKSLSLSPGK |
| 31905-44AA Light Chain | 5 | DIVMTQSPDSLAVSLGERVTMNCKSSQSLLNAGNQKNY LTWYQQKPGQPPKLLFYWASTRESGVPDRFSGSGSGTDF TLTISSVQAEDVAVYHCQNAYYYPYTFGGGTKLEIKRTV AAPSVFIFPPSDEQLKSGTASVVCLLNNFYPREAKVQWK VDNALQSGNSQESVTEQDSKDSTYSLSSTLTLSKADYEK |
| | | HKVYACEVTHQGLSSPVTKSFNRGEC |
| 6# AA Heavy Chain | 1 | QVQLVQSGAEVKKPGASVKVSCKASGYIFTNYWIHWV RQAPGQGLEWMGRIYPGTGNTYYNEKFTGRVTMTRDT STSTVYMELSSLRSEDTAVYYCAREGYGKGNSMDYWG QGTTVTVSSASTKGPSVFPLAPSSKSTSGGTAALGCLVK DYFPEPVTVSWNSGALTSGVHTFPAVLQSSGLYSLSSVVT VPSSSLGTQTYICNVNHKPSNTKVDKKVEPKSCDKTHTC PPCPAPEAAGGPSVFLFPPKPKDTLMISRTPEVTCVVVDV SHEDPEVKFNWYVDGVEVHNAKTKPREEQYNSTYRVV SVLTVLHQDWLNGKEYKCKVSNKALPAPIEKTISKAKG QPREPQVYTLPPSRDELTKNQVSLTCLVKGFYPSDIAVEW ESNGQPENNYKTTPPVLDSDGSFFLYSKLTVDKSRWQQG NVFSCSVMHEALHNHYTQKSLSLSPGK |
| 6# AA Light Chain | 5 | DIVMTQSPDSLAVSLGERVTMNCKSSQSLLNAGNQKNY LTWYQQKPGQPPKLLFYWASTRESGVPDRFSGSGSGTDF TLTISSVQAEDVAVYHCQNAYYYPYTFGGGTKLEIKRTV AAPSVFIFPPSDEQLKSGTASVVCLLNNFYPREAKVQWK VDNALQSGNSQESVTEQDSKDSTYSLSSTLTLSKADYEK HKVYACEVTHQGLSSPVTKSFNRGEC |
| h 160C9AA Variable Region of Heavy Chain VH | 6 | EVKLVESGGGLVQPGGSLKLSCAASGFTFNTYAMNWVR QAPGKGLEWVARIRSKYNNYATYYADSVKDRFTISRDD AKNTLYLQMNNLRTEDTAVYYCVRHGNFGNSYVSWFA YWGQGTLVTVSS |
| h 160C9AA Variable Region of Light Chain VL | 7 | QAVVTQEPSLTVSPGGTVTLTCRSSTGAVTTSNYANWVQ QKPGQAPRGLIGGTNKRAPGTPARFSGSLLGGKAALTLS GAQPEDEAEYYCALWYSNLWVFGGGTKLTVL |
| scFv of h 160C9AA | 8 | AQAVVTQEPSLTVSPGGTVTLTCRSSTGAVTTSNYANWV QQKPGQAPRGLIGGTNKRAPGTPARFSGSLLGGKAALTL SGAQPEDEAEYYCALWYSNLWVFGGGTKLTVLGGGGS GGGGSGGGGSEVKLVESGGGLVQPGGSLKLSCAASGFT FNTYAMNWVRQAPGKGLEWVARIRSKYNNYATYYADS VKDRFTISRDDAKNTLYLQMNNLRTEDTAVYYCVRHGN FGNSYVSWFAYWGQGTLVTVSS |
| h 160C9AA Heavy Chain | 9 | EVKLVESGGGLVQPGGSLKLSCAASGFTFNTYAMNWVR QAPGKGLEWVARIRSKYNNYATYYADSVKDRFTISRDD AKNTLYLQMNNLRTEDTAVYYCVRHGNFGNSYVSWFA YWGQGTLVTVSSASTKGPSVFPLAPSSKSTSGGTAALGC LVKDYFPEPVTVSWNSGALTSGVHTFPAVLQSSGLYSLSS VVTVPSSSLGTQTYICNVNHKPSNTKVDKKVEPKSCDKT HTCPPCPAPEAAGGPSVFLFPPKPKDTLMISRTPEVTCVV VDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRW QQGNVFSCSVMHEALHNHYTQKSLSLSPGK |
| h 160C9AA Light Chain | 10 | QAVVTQEPSLTVSPGGTVTLTCRSSTGAVTTSNYANWVQ QKPGQAPRGLIGGTNKRAPGTPARFSGSLLGGKAALTLS GAQPEDEAEYYCALWYSNLWVFGGGTKLTVLGQPKAN PTVTLFPPSSEELQANKATLVCLISDFYPGAVTVAWKADS SPVKAGVETTTPSKQSNNKYAASSYLSLTPEQWKSHRSY SCQVTHEGSTVEKTVAPTEC |
| 6# AA Heavy Chain HCDR1 | 11 | NYWIH |
| 6# AA Heavy Chain HCDR2 | 12 | RIYPGTGNTYYNEKFTG |
| 6# AA Heavy Chain HCDR3 | 13 | EGYGKGNSMDY |
| 6# AA Light Chain LCDR1 | 14 | KSSQSLLNAGNQKNYLT |
| 6# AA Light Chain LCDR2 | 15 | WASTRES |
| 6# AA Light Chain LCDR3 | 16 | QNAYYYPYT |
| h 160C9AA Heavy Chain HCDR1 | 17 | TYAMN |
| h 160C9AA Heavy Chain HCDR1 | 18 | RIRSKYNNYATYYADSVKD |
| h 160C9AA Heavy Chain HCDR1 | 19 | HGNFGNSYVSWFAY |
| h 160C9AA Light Chain LCDR1 | 20 | RSSTGAVTTSNYAN |
| h 160C9AA Light Chain LCDR2 | 21 | GTNKRAP |
| h 160C9AA Light Chain LCDR3 | 22 | ALWYSNLWV |
| 6# AA Variable Region of Heavy Chain | 23 | QVQLVQSGAEVKKPGASVKVSCKASGYIFTNYWIHWV RQAPGQGLEWMGRIYPGTGNTYYNEKFTGRVTMTRDT STSTVYMELSSLRSEDTAVYYCAREGYGKGNSMDYWG |
| VH | | QGTTVTVSS |
| 6# AA Variable Region of Light Chain VL | 24 | DIVMTQSPDSLAVSLGERVTMNCKSSQSLLNAGNQKNY LTWYQQKPGQPPKLLFYWASTRESGVPDRFSGSGSGTDF TLTISSVQAEDVAVYHCQNAYYYPYTFGGGTKLEIK |

### Example 2 Construction of anti-CD3-CLDN18.2 bispecific antibody and transient expression in eukaryotic cells

Transfection-grade expression plasmids were prepared by cloning the gene fragments of interest from the heavy and light chains of the above bispecific antibody molecules into PTT5 expression vectors, respectively. The plasmid was inoculated in serum-free medium to culture HEK293E or Expi293 cells and cultured on a shaker at 37°C and 8% CO₂. Supernatants were collected after 6 days of cell culture and the final purified bispecific antibody was subjected to SDS-PAGE purity analysis and A280 concentration determination.

### Example 3 Affinity detection assay for anti-CD3-CLDN18.2 bispecific antibodies

### A. Detection of affinity of anti-CD3-CLDN18.2 bispecific antibodies to cells expressing hCLDN18.2 and hCD3

The binding situation of the anti-CD3-CLDN18.2 bispecific antibodies to stably-transfected HEK293 cells expressing hCLDN18.2 (HEK293-hCLDN18.2) and T lymphocytes naturally expressing hCD3 (Jurkat) were detected by using FACS. HEK293-hCLDN18.2 or Jurkat cells were collected and re-suspended with FACS buffer (PBS + 1% BSA + 0.5 mM EDTA), after adjusting the cell concentration, 1E5 cells per well were added into a 96-well plate, and then the antibody after gradient dilution was added according to the preset concentration, wherein the negative control was human IgGlAA (Negative-IgG1AA), the positive control of CLDN18.2 was antibody 6#AA, and the positive control of CD3 was antibody h160C9AA. Following incubation in a 4°C shaking table for 2 h, the mixture was centrifugally washed twice with FACS buffer, added with fluorescently labeled anti-human IgG secondary antibody, 100 µL per well; after incubation in a 4°C shaking table for 1 h, the mixture was centrifugally washed twice with FACS buffer, the cells were filtered to a 96-well plate, and then the prepared sample was detected on a flow cytometer; the mean fluorescence intensity (hereinafter referred to as MFI) for each concentration was calculated by software, and then the median effective concentration (hereinafter referred to as EC₅₀) and the top mean fluorescence intensity (Top MFI) were calculated by GraphPad software, and the results were shown in Table 3.

**Table 3 Affinity of anti-CD3-CLDN18.2 bispecific antibodies to hCLDN18.2 and hCD3, respectively**

| Molecular Number | HEK293-hCLDN18.2 | | Jurkat | |
|---|---|---|---|---|
| | EC₅₀ (nM) | Top mean fluorescence intensity (Top MFI) | EC₅₀ (nM) | Top mean fluorescence intensity (Top MFI) |
| 31905-38AA | 0.98 | 24200 | 14.08 | 2025 |
| 31905-44AA | 1.18 | 31000 | 9.3 | 1698 |
| 6#AA | 0.95 | 18600 | N/A | 26 |
| h 160C9AA | 0 | 897 | 0.13 | 2020 |
| Negative-IgG 1AA | 0 | 359 | 0 | 110 |

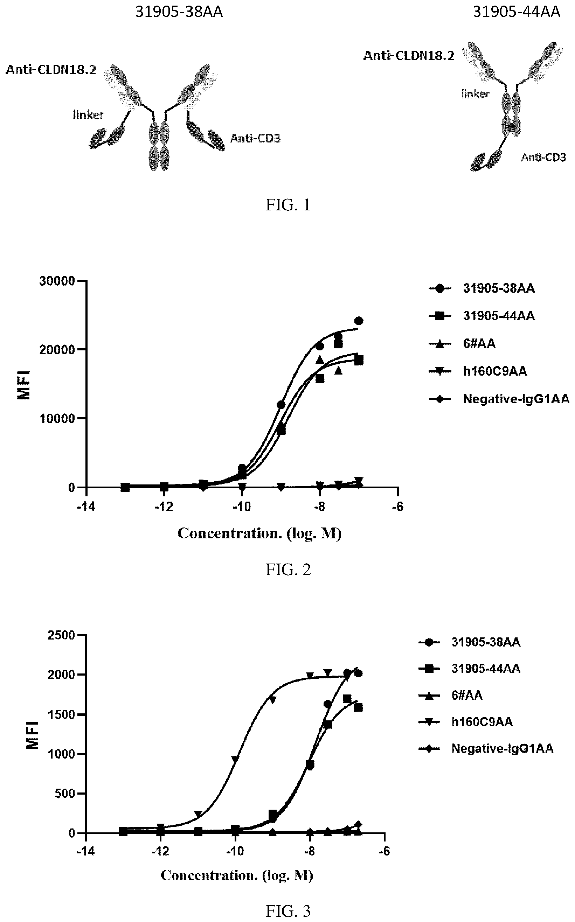

Table 3 and FIGs. 2-3 showed the affinity results for the anti-CD3-CLDN18.2 bispecific antibodies of the present disclosure and the reference antibodies (6# AA and h160C9AA) to HEK293-hCLDN18.2 cells and Jurkat cells, respectively. The results showed that: the bispecific antibodies of the present disclosure bound to HEK293-hCLDN18.2 cells with a top mean fluorescence intensity of between 24200 and 31000, while the anti-CLDN18.2 reference antibody 6#AA bound to HEK293-hCLDN18.2 under the same reaction conditions with a top mean fluorescence intensity of 18600, and the anti-CD3 reference antibody h160C9AA bound to HEK293-hCLDN18.2 under the same reaction conditions with a top mean fluorescence intensity of only 897. The bispecific antibodies of the present disclosure bound to HEK293-hCLDN18.2 with a median effective concentration (EC₅₀) of 0.98-1.18, the anti-CLDN18.2 reference antibody 6#AA bound to HEK293-hCLDN18.2 with a median effective concentration (EC₅₀) of 0.95 nM under the same reaction conditions, and the anti-CD3 reference antibody h160C9AA bound to HEK293-hCLDN18.2 with a median effective concentration (EC₅₀) of 0 nM under the same reaction conditions.

The anti-CD3-CLDN18.2 bispecific antibodies of the present disclosure bound to CD3-highly expressed Jurkat cells with a top mean fluorescence intensity between 1698 and 2025, whereas the anti-CD3 reference antibody h160C9AA bound to Jurkat with a top mean fluorescence intensity of 2020 under the same reaction conditions. The bispecific antibodies of the present disclosure bound to Jurkat with a median effective concentration (EC₅₀) between 9.3 and 14.08 and the anti-CD3 reference antibody h160C9AA bound to Jurkat with a median effective concentration (EC₅₀) of 0.13 nM under the same reaction conditions. It can be seen therefrom that the binding capacity of the bispecific antibodies of the present disclosure to the hCLDN18.2 antigen is substantially consistent with that of the CLDN18.2 reference monoclonal antibody 6#AA, without significant difference; the binding capacity of the bispecific antibodies to the hCD3 antigen was weaker than that of CD3 reference antibody h160C9AA.

### B. In vitro binding affinity and kinetics of anti-CD3-CLDN18.2 bispecific antibodies

The affinity and kinetic properties of the bispecific antibodies to human CD3 E&D proteins were determined by surface plasmon resonance (SPR) using a Biacore 8K instrument. The human CD3 E&D protein was immobilized on the experimental channel of CM5 chip by amino coupling, then the antibody to be tested was subjected to doubling dilution to serial concentrations, flowed through the surfaces of an experimental channel and a reference channel successively for binding, followed by dissociation, the binding and dissociation curve of each sample was obtained, and the results were analyzed and evaluated by Biacore Insight Evaluation software.

The bispecific antibodies of the present disclosure were diluted to 400 nM with 1×HBS-EP+(10 mM HEPES, pH 7.4, 150 mM NaCl, 3 mM EDTA, 0.05% P20) running buffer, then subjected to doubling dilution to 1.56 nM with this buffer, thus obtaining antibody solutions in a range of concentrations from 400 nM to 1.56 nM. At the end of each experiment, the chip was regenerated by washing with 3M MgCl₂ solution at a flow rate of 30 µL/min for 30 s to remove captured antibody together with antigen. The raw data were analyzed using Biacore Insight Evaluation Software (version 2.0.15.12933) with a 1:1 fit model and affinity and kinetic experimental data of the resulting bispecific antibodies were shown in Table 4. The results showed that the bispecific antibodies had high affinity to humanized CD3 E&D protein, and the affinity of 31905-44AA molecule to humanized CD3 E&D protein was weaker than that of 31905-38AA molecule.

**Table 4 Binding affinity and kinetics of anti-CD3-CLDN18.2 bispecific antibodies to humanized CD3 E&D protein**

| Molecular Number | Antigen | Binding rate kₐ (1/M^{∗}s) | Dissociation rate k_{d} (1/s) | Affinity K_{D} (M) |
|---|---|---|---|---|
| 31905-3 8AA | Humanized CD3 E&D | 7.82E+05 | 7.66E-04 | 9.78E-10 |
| 31905-44AA | Humanized CD3 E&D | 2.04E+05 | 3.65E-03 | 1.79E-08 |

### C. Binding selectivity of anti-CD3-CLDN18.2 bispecific antibodies

The binding situation of the anti-CD3-CLDN18.2 bispecific antibodies of the present disclosure, the anti-CLDN18.2 reference antibody 6#AA, and the anti-CD3 reference antibody h160C9AA binding to stably-transfected HEK293 cells expressing hCLDN18.1 (HEK293-hCLDN18.1), stably-transfected HEK293 cells expressing mCLDN18.2 (HEK293-mCLDN18.2), stably-transfected HEK293 cells expressing mCLDN18.1 (HEK293-mCLDN18.1), and stably-transfected HEK293 cells expressing cynoCLDN18 (HEK293-cynoCLDN18) were detected by using FACS, respectively. HEK293-hCLDN18.1, HEK293-mCLDN18.2, HEK293-mCLDN18.1, and HEK293-cynoCLDN18 cells were prepared and re-suspended with FACS buffer (PBS + 1% BSA + 0.5 mM EDTA); after adjusting the cell concentration, 1E5 cells per well were added into a 96-well plate, and then the antibody after gradient dilution was added according to the preset concentration, wherein the negative control was Negative-IgG1AA, the positive control of CLDN18.2 was antibody 6#AA, and the positive control of CD3 was antibody h160C9AA. Following incubation in a 4°C shaking table for 2 h, the mixture was centrifugally washed twice with FACS buffer, added with fluorescently labeled anti-human IgG secondary antibody, 100 µL per well; after incubation in a 4°C shaking table for 1 h, the mixture was centrifugally washed twice with FACS buffer, the cells were filtered to a 96-well plate, and then the prepared sample was detected on a flow cytometer; the mean fluorescence intensity (hereinafter referred to as MFI) for each concentration was calculated by software, and then the median effective concentration (hereinafter referred to as EC₅₀) and the top mean fluorescence intensity (Top MFI) were calculated by GraphPad software, and the results were shown in Table 5.

**Table 5 Binding of anti-CD3-CLDN18.2 bispecific antibodies to hCLDN18.1, mCLDN18.2, mCLDN18.1, and cynoCLDN18, respectively**

| Molecular Number | HEK293-mCLDN1 8.2 | | HEK293-cynoCLD N18 | | HEK293-hC LDN18.1 | HEK293-mCL DN18.1 |
|---|---|---|---|---|---|---|
| | EC₅₀ (nM) | MFI (Max) | EC₅₀ (nM) | MFI (Max) | Binding or not (+/-) | Binding or not (+/-) |
| 31905-38 AA | 1.13 | 37300 | 0.58 | 28600 | - | - |
| 31905-44 AA | 1.18 | 51400 | 0.84 | 41800 | - | - |
| 6#AA | 0.90 | 36000 | 0.62 | 39500 | - | - |
| h160C9A A | 0 | 543 | 0 | 1045 | - | - |
| Negative-I gG1AA | 0 | 75 | N/A | 136 | - | - |

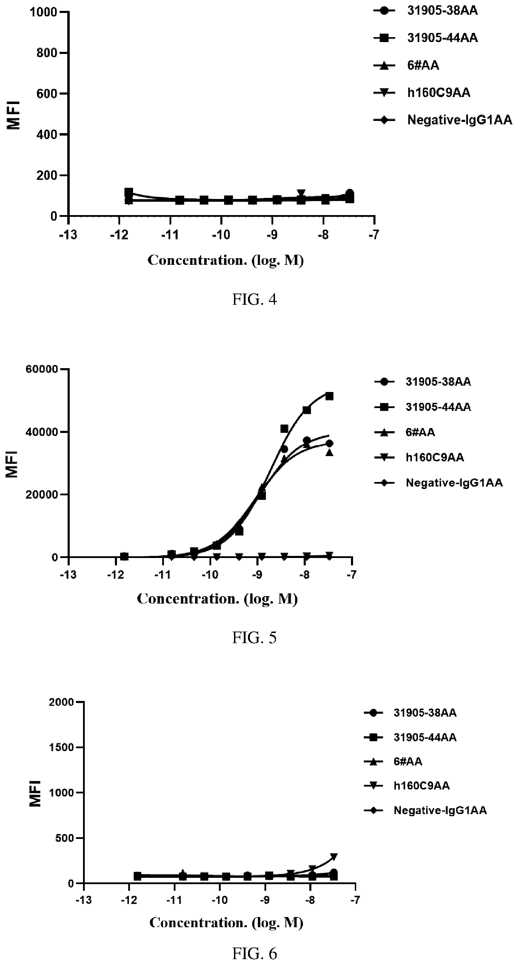

Table 5 and FIGs. 4-7 showed the affinity results of the anti-CD3-CLDN18.2 bispecific antibodies of the present disclosure, the anti-CLDN18.2 reference antibody 6#AA, and the anti-CD3 reference antibody h160C9AA to HEK293-hCLDN18.1 cells, HEK293-mCLDN18.2 cells, HEK293-mCLDN18.1 cells, and HEK293-cynoCLDN18 cells, respectively. The results showed that: the bispecific antibodies of the present disclosure was the same as the reference antibody 6#AA, both of which bound to mCLDN18.2 and cynoCLDN18, the bispecific antibodies bound to HEK293-mCLDN18.2 with a top mean fluorescence intensity of between 37300 and 51400, the reference antibody 6#AA bound to HEK293-mCLDN18.2 under the same reaction conditions with a top mean fluorescence intensity of 36000, and the reference antibody h160C9AA bound to HEK293-mCLDN18.2 under the same reaction conditions with a top mean fluorescence intensity of only 543; the bispecific antibodies of the present disclosure had a median effective concentration (EC₅₀) of between 1.13 and 1.18; the reference antibodies 6#AA and h160C9AA had median effective concentrations (EC₅₀) of 0.90 nM and 0 nM, respectively. Furthermore, the experimental results showed that the bispecific antibodies of the present disclosure bound to HEK293-cynoCLDN18 with a top mean fluorescence intensity of between 28600 and 41800, and the reference antibodies 6#AA and h160C9AA bound to HEK293-cynoCLDN18 under the same reaction conditions with a top mean fluorescence intensity of 39500 and 1045, respectively; the bispecific antibodies had a median effective concentration (EC₅₀) of between 0.58 and 0.84; the reference antibodies 6#AA and h160C9AA had median effective concentrations (EC₅₀) of 0.62 nM and 0 nM, respectively. Further study results showed that neither the bispecific antibodies of the present disclosure nor the reference antibody bound to hCLDN18.1 and mCLDN18.1. It can be seen therefrom that the binding of the bispecific antibodies of the present disclosure to mCLDN18.2 and cynoCLDN18 was substantially identical to the reference antibody 6#AA: they bound to human and murine CLDN18.2 and cynoCLDN18 specifically, but not to human and murine CLDN18.1, with a good selectivity.

### Example 4 In vitro functional assay for anti-CD3-CLDN18.2 bispecific antibodies

### A. T-cell dependent cellular cytotoxicity (TDCC)

With HEK293-hCLDN18.2 as target cells and PBMC cells of healthy person as effector cells, and the release of lactate dehydrogenase (LDH) was detected by the cytotoxicity assay reagent (Roche) and used as an indicator of cell killing effect.

HEK293-hCLDN18.2 cells were collected by centrifugation, the supernatant was discarded, the cell was re-suspended with cell buffers (RMPI1640+1%FBS+1%P/S) to adjust the cell density, then 50 µL of cells were transferred to a 96-well plate according to the cell number of 1E4 per well overnight; after cell attachment, 50 µL of the antibody with different concentration gradients prepared in advance as well as the control sample working solution were added, and then 50 µL of pre-prepared PBMC cells were supplemented according to the cell number of effector cells:target cells = 20:1, i.e., 1E5 per well. The cells were incubated in a cell culture incubator (37°C, 5% CO₂) for about 24 h. After the incubation, the 96-well assay plate was removed and centrifuged for 10 min at 1500 rpm, 50 µL of supernatant was pipetted carefully and transferred into a new 96-well assay plate, isovolumetric LDH detection working solution was added, the plate was incubated at room temperature for about 20 min, then was detected on a microplate reader, wherein a detection wavelength was 492 nm, and a reference wavelength was 650 nm.

The percentage of cell lysis caused by TDCC effect was calculated using the following formula:
%cell lysis = 100% × (sample release-target cell/effector cell release)/(maximum release-target cell release), wherein the maximum release was the absorbance value produced in the wells of target cells treated with Triton X-100, the target cell/effector cell mixed release was the absorbance value produced in the wells of target cells and effector cell mixture, and the target cell release was the absorbance value produced in the wells containing only target cells, the samples release was the absorbance values produced in the wells of antibodies, target cells and effector cells mixture, and EC₅₀ and maximal lysis were calculated by GraphPad software, and the results were shown in Table 6.

### B. T cell activation assay

T cell activation assay was mainly identified by determining the cell activation marker CD25⁺CD69⁺%. The cells after TDCC assay in 96-well plate were collected, centrifuged and washed twice with FACS buffer (PBS + 1% BSA + 0.5 mM EDTA), then a certain amount of anti-human APC-CD3, anti-human PE-CD25, and anti-human BV421-CD69 mixture was added, and the mixture was incubated at 4°C for 1 h. After centrifugation and washing twice with FACS buffer, the cells were filtered into a new 96-well plate, and then the prepared samples were detected on a flow cytometer. The percentage of CD25⁺CD69⁺ in CD3⁺ T cells was calculated by software, and then the median effective concentration (hereinafter referred to as EC₅₀) and the highest percentage (CD25⁺CD69⁺%) were calculated by GraphPad software. The results were shown in Table 6.

**Table 6 TDCC activity and T cell activation of anti-CD3-CLDN18.2 bispecific antibodies**

| Molecular Number | TDCC activity | | T cell activation | |
|---|---|---|---|---|
| | EC₅₀ (nM) | Maximum lysis (%) | EC₅₀ (nM) | CD25⁺CD69⁺% |
| 31905-38AA | 0.13 | 54.62% | 0.022 | 38.86% |
| 31905-44AA | 0.54 | 30.03% | 0.049 | 33.43% |
| 6#AA | 1.45 | 16.34% | 0 | 3.18% |
| h160C9AA | 0.32 | 3.12% | 0 | 4.2% |
| Negative-IgG 1AA | 0 | 1.56% | 0 | 0% |

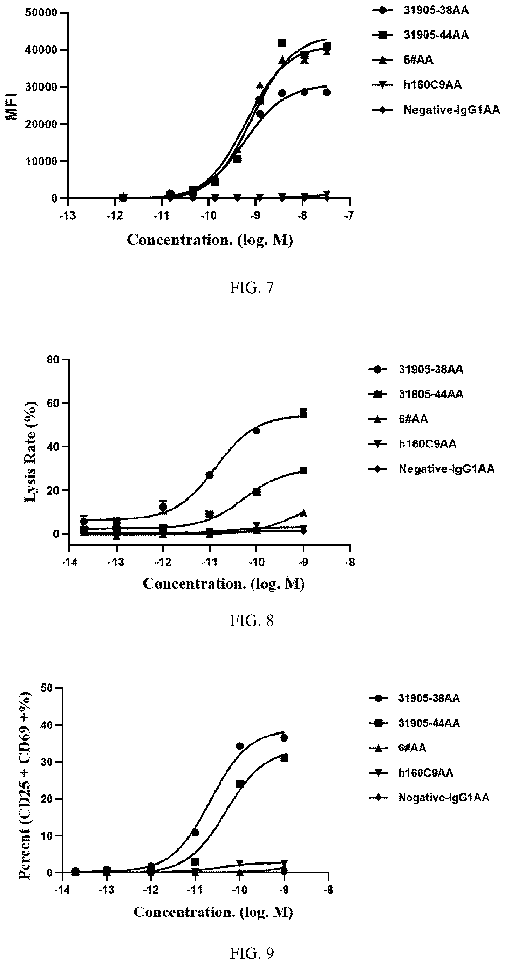

Table 6 and FIGs. 8-9 showed the TDCC activity and T cell activation results of the anti-CD3-CLDN18.2 bispecific antibodies of the present disclosure on HEK293-hCLDN18.2 cells. The results showed that a maximum TDCC effect of the bispecific antibodies of the present disclosure on HEK293-hCLDN18.2 cells is between 30.03% and 54.62%, and a maximum TDCC effect of the anti-CLDN18.2 reference antibody 6#AA on HEK293-hCLDN18.2 cells under the same reaction conditions is 16.34% respectively; a 50% TDCC effect concentration (EC₅₀) produced by the bispecific antibodies is between 0.13 nM and 0.54 nM, and a 50% TDCC effect concentration (EC50) produced by the reference antibody 6#AA under the same reaction conditions is 1.45 nM.

The maximum T cell activation effect of the bispecific antibodies of the present disclosure on HEK293-hCLDN18.2 cells is between 33.43% and 38.86%, and the maximum T cell activation effect of the reference antibody 6#AA and h160C9AA on HEK293-hCLDN18.2 cells under the same reaction conditions is only 3.18% and 4.2%, respectively; a 50% T cell activation concentration (EC₅₀) produced by the bispecific antibodies is between 0.022 nM and 0.049 nM, and a 50% T cell activation concentration (EC₅₀) produced by the reference antibodies 6#AA and h160C9AA is 0 under the same reaction conditions. The above results demonstrated that the bispecific antibodies of the present disclosure have strong TDCC activity and T cell activation effects, which are target cell dependent.

### C. Cytokine quantitation assay

Cytokines quantitation assay (including INF-γ, IL-2, IL-6, and TNF-α) were mainly detected by using Elisa Kit (Biolegend). The supernatant after centrifugation was collected, diluted according to a set ratio, and detected with the Kit by referring to Kit Protocol for the specific methods. The detection was performed on a microplate reader, with a detection wavelength of 450 nm and a reference wavelength of 650 nm. The EC₅₀ and maximum concentrations were calculated by the GraphPad software and the results were shown in Tables 7 and 8.

**Table 7 Secretions of INF-γ and IL-2 in T cells induced by anti-CD3-CLDN18.2 bispecific antibodies**

| Molecular Number | INF-γ secretion | | IL-2 secretion | |
|---|---|---|---|---|
| | EC₅₀ (nM) | Concentration (pg/mL) | EC₅₀ (nM) | Concentration (pg/mL) |
| | | (Max) | | (Max) |
| 31905-38AA | 0.012 | 769.5 | 0.013 | 201.9 |
| 31905-44AA | 0.033 | 260.5 | 0.05 | 60.25 |
| 6#AA | 0 | 0 | 0 | 0 |
| h160C9AA | 0 | 0 | 0 | 0 |
| Negative-IgG1AA | 0 | 0 | 0 | 0 |

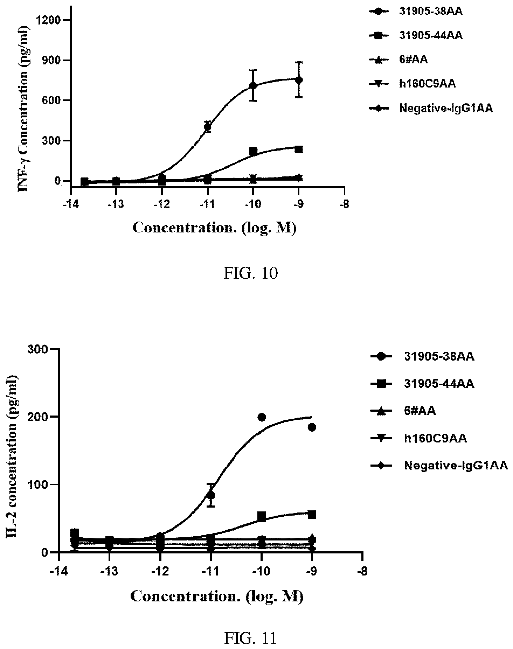

Table 7 and FIGs. 10-11 showed the results of INF-γ and IL-2 secreted in T cells induced by the anti-CD3-CLDN18.2 bispecific antibodies of the present disclosure in the presence of HEK293-hCLDN18.2 cells. The results showed that the bispecific antibodies-induced T cells secreted INF-γ at a maximum level of between 260.5 pg/mL and 769.5 pg/mL; and the bispecific antibodies induced 50% INF-γ secretion at a concentration (EC₅₀) of between 0.012 nM and 0.033 nM. The reference antibodies 6#AA and h160C9AA did not significantly induce INF-γ secretion under the same reaction conditions.

The bispecific antibodies-induced T cells secreted INF-γ at a maximum level of between 60.25 pg/mL and 201.9 pg/mL; and the bispecific antibodies induced 50% INF-γ secretion at a concentration (EC₅₀) of between 0.013 nM and 0.05 nM. Reference antibodies 6#AA and h160C9AA did not significantly induce IL-2 secretion under the same reaction conditions.

**Table 8 Secretions of IL-6 and TNF-α in T cells induced by anti-CD3-CLDN18.2 bispecific antibodies**

| Molecular Number | IL-6 secretion | | TNF-α secretion | |
|---|---|---|---|---|
| | EC₅₀ (nM) | Concentration (pg/mL) | EC₅₀ (nM) | Concentration (pg/mL) |
| | | (Max) | | (Max) |
| 31905-38AA | 0.005 | 21942 | 0.01 | 664.6 |
| 31905-44AA | 0.16 | 12718 | 1.3 | 365.9 |
| 6#AA | 0 | 0 | 0 | 0 |
| h160C9AA | 2.9 | 84209 | 5.9 | 3175 |
| Negative-IgG1AA | 0 | 0 | 0 | 0 |

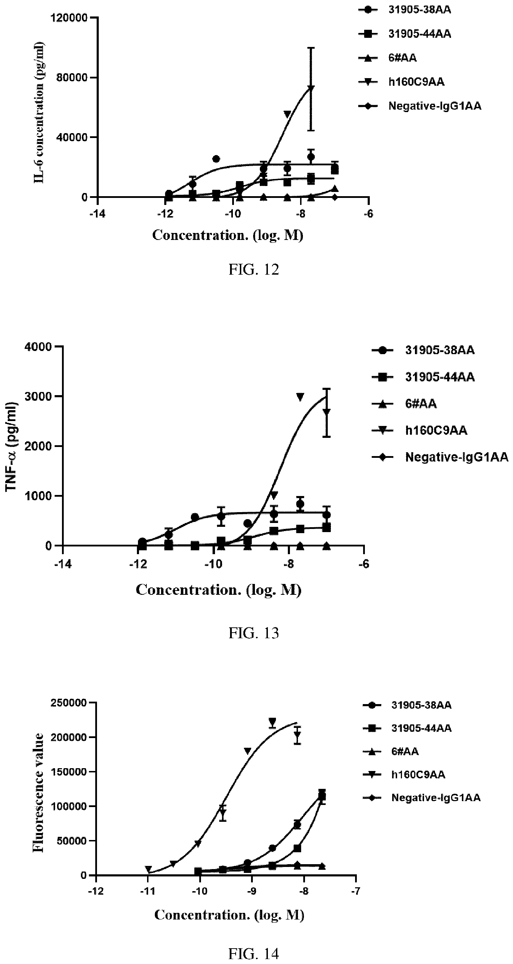

Table 8 and FIGs. 12-13 showed the results of secretions of IL-6 and TNF-α in T cells induced by the anti-CD3-CLDN18.2 bispecific antibodies of the present disclosure in the presence of HEK293-hCLDN18.2 cells. The results showed that the bispecific antibodies of the present disclosure induced T cells to secrete IL-6 at maximum level of between 12718 pg/mL and 21942 pg/mL, and the reference antibody h160C9AA induced T cells to secrete IL-6 at a maximum level of 84209 pg/mL under the same reaction conditions; the bispecific antibodies induced a 50% IL-6 secretion at a concentration (EC₅₀) between 0.005 nM and 0.16 nM, and the reference antibody h160C9AA induced 50% IL-6 secretion at a concentration (EC₅₀) of 2.9 nM under the same reaction conditions. However, the reference antibody 6#AA did not significantly induce IL-6 secretion under the same reaction conditions.

The anti-CD3-CLDN18.2 antibody of the present disclosure induced T cells to secrete TNF-α at a maximum level of between 365.9 pg/mL and 664.6 pg/mL, and the reference antibody h160C9AA induced T cells to secrete TNF-α at a maximum level of 3175 pg/mL under the same reaction conditions; the bispecific antibodies induced a 50% TNF-α secretion at a concentration (EC₅₀) between 0.01 nM and 1.3 nM, and the reference antibody h160C9AA induced 50% TNF-α secretion at a concentration (EC₅₀) of 5.9 nM under the same reaction conditions. However, the reference antibody 6#AA did not significantly induce TNF-α secretion under the same reaction conditions.

These results further demonstrated that the anti-CD3-CLDN18.2 antibodies of the present disclosure have strong T cell activation. In the presence of target cells, induction of INF-γ and IL-2 secretions was stronger than that of the anti-CD3 reference antibody h160C9AA, and induction of IL-6 and TNF-α secretions was weaker than that of h160C9AA.

### D. Luciferase reporter assay

Jurkat-NFAT cell line was constructed to stably express luciferase, the gene of which was regulated by NFAT element. The NFAT response element was regulated by CD3 receptors on the surface of Jurkat cells and activated via the stimulation of CD3 receptors to express luciferase, so this system was used to mimic the activation effect of CD3 antibodies on T cells at the cellular level.

The Jurkat-NFAT cells were diluted and mixed with culture medium (RMPI1640 + 1% FBS + 1% P/S), the mixture was inoculated into a designated well plate at 50 µL per well according to the cell number of 5E4 per well, then 50 µL of test antibodies which had been diluted with culture medium was added and incubated with the cells for about 4-6 h in a 5% CO₂ incubator at 37°C, 50 µL of detection reagent Bright-Glo luciferase assay reagent was added, the mixture was incubated away from light 5 min at room temperature, the chemiluminescence signal value was read on PerkinElmer Envision, and the signal inhibition EC₅₀ value was fit by GraphPad Prism.

**Table 9 Expression of Jurkat-NFAT reporter gene by anti-CD3-CLDN18.2 bispecific antibodies**

| Molecular Number | EC₅₀ (nM) | Fluorescence value (Max) |
|---|---|---|
| 31905-38AA | 7.62 | 116960 |
| 31905-44AA | 123.6 | 113700 |
| 6#AA | 0 | 0 |
| h160C9AA | 0.32 | 220000 |
| Negative-IgG1AA | 0 | 0 |

Table 9 and FIG. 14 showed the results of luciferase expression levels of the anti-CD3-CLDN18.2 bispecific antibodies of the present disclosure against the Jurkat-NFAT reporter gene. The results showed that: the maximum luciferase expression of the bispecific antibodies of the present disclosure against the Jurkat-NFAT reporter gene was between 113700 and 116960, the maximum luciferase expression of the reference antibody h160C9AA against the Jurkat-NFAT reporter gene was 220000, and the reference antibody 6#AA had no effect on the maximum luciferase expression of the Jurkat-NFAT reporter gene.

The bispecific antibodies of the present disclosure produced 50% luciferase expression at a concentration (EC₅₀) of between 7.62 and 123.6nM and the reference antibody h160C9AA antibody produced 50% luciferase expression at a concentration (EC₅₀) of 0.32 nM, demonstrating that the bispecific antibodies of the present disclosure have a lower activating effect than the CD3 reference antibody and the CD3 bivalent antibody (31905-38AA) has a higher activating effect than the CD3 monovalent antibody (31905-44AA).

### E. NK-cell mediated cellular cytotoxicity (ADCC)

With HEK293-hCLDN18.2 as target cells and NK cells isolated from healthy human PBMC as effector cells, and the release of lactate dehydrogenase (LDH) was detected by the cytotoxicity assay reagent (Roche) and used as an indicator of cell killing effect. HEK293-hCLDN18.2 cells were centrifugated and collected, the supernatant was discarded, the cell was re-suspended with ADCC buffers (RMPI1640+1%FBS+1%P/S) to adjust the cell density, then 50 µL of cells were transferred to a 96-well plate according to the cell number of 1E4 per well overnight; after cell attachment, 50 µL of the antibody with different concentration gradients prepared in advance as well as the control sample working solution were added, and then 50 µL of pre-isolated NK cells were supplemented according to the cell number of effector cells:target cells = 5:1, i.e., 5E4 per well. The cells were incubated in a cell culture incubator (37°C, 5% CO₂) for about 24 h. After the incubation, the 96-well assay plate was removed and centrifuged for 10 min at 1500 rpm, 50 µL of supernatant was pipetted carefully and transferred into a new 96-well assay plate, isovolumetric LDH detection working solution was added, the plate was incubated at room temperature for about 20 min, then was detected on a microplate reader, wherein a detection wavelength was 492 nm, and a reference wavelength was 650 nm.

The percentage of cell lysis caused by ADCC effect was calculated using the following formula:
%cell lysis = 100% × (sample release-target cell release/effector cell release)/(maximum release-target cell release), wherein the maximum release was the absorbance value produced in the wells of target cells treated with Triton X-100, the target cell/effector cell mixed release was the absorbance value produced in the wells of target cells and effector cell mixture, and the target cell release was the absorbance value produced in the wells containing only target cells, the samples release was the absorbance values produced in the wells of chimeric antibody, target cells and effector cells mixture, and EC₅₀ and maximal lysis were calculated by GraphPad software, and the results were shown in Table 10.

**Table 10 ADCC activity of anti-CD3-CLDN18.2 bispecific antibodies**

| Molecular Number | ADCC activity | |
|---|---|---|
| | EC₅₀ (nM) | Maximum lysis (%) |
| 31905-38AA | 0.501 | 19.95% |
| 31905-44AA | 0.532 | 7.03% |
| 6#AA | 0.771 | 6.92% |
| h160C9AA | 0 | 0 |
| Negative-IgG1AA | 0 | 0 |

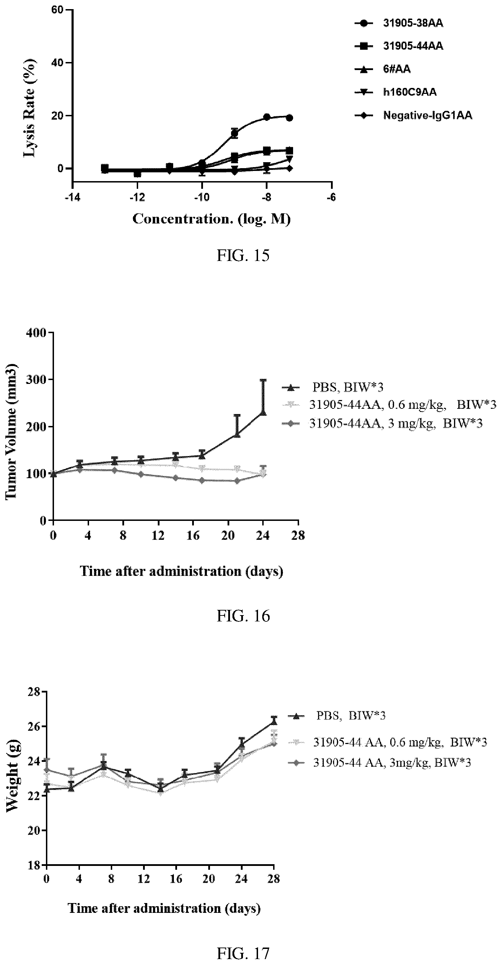

Table 10 and FIG. 15 showed the results of ADCC activity of anti-CD3-CLDN18.2 bispecific antibodies of the present disclosure on HEK293-hCLDN18.2 cells. The results showed that: the maximum ADCC effect of the bispecific antibodies of the present disclosure on HEK293-hCLDN18.2 cells was between 7.03% and 19.95%, and the ADCC effect of reference antibodies 6#AA and h160C9AA under the same reaction condition was 6.92% and 0%, respectively. The bispecific antibodies of the present disclosure produced a 50% ADCC effect at a concentration (EC₅₀) of between 0.501 nM and 0.532 nM, and 6#AA produced a 50% ADCC effect at a concentration (EC₅₀) of 0.771 nM under the same reaction conditions. The above results demonstrated that the ADCC effect of the anti-CD3-CLDN18.2 bispecific antibodies of the present disclosure was comparable to that of the anti-CLDN18.2 reference antibody 6#AA, whereas the CD3 reference antibody h160C9AA had no ADCC effect.

### Example 5 In vivo pharmacodynamic experiments for anti-CD3-CLDN18.2 bispecific antibodies

Peripheral blood of normal person was drawn, and human PBMC were isolated by density gradient centrifugation and counted. The isolated PBMC was then added to Mitomycin C-treated HEK293-hCLDN18.2 cells, and the mixture was co-cultured for 6 days using RPMI-1640 medium (containing IL-2 and 10% FBS). Six days later, PBMC was harvested, and PBMC cells were mixed inoculated in NCG mice (GemPharmatech Co., Ltd.) with freshly digested HEK293-hCLDN18.2 cells (Shanghai Qilu Pharmaceutical Research and Development Center Ltd.) to construct a humanized HEK293-hCLDN18.2 model. The experimental animals were housed in an independent ventilated box with constant temperature and humidity, wherein temperature of the housing room was 20.0-26.0°C, the humidity was 40-70%, the ventilation was 10-20 times/h, and the day and night (light: dark) alternation period was 12h/12h.

The experiment was divided into PBS control group, 0.6 mg/kg 31905-44 AA treatment group, and 3 mg/kg 31905-44 AA treatment group. Five mice in each group were administrated via intraperitoneal injection, twice a week for 6 times (see Table 11). Animals were monitored daily for behavioral performance for 24 days after administration. Throughout the experiment, tumor long and wide diameters were measured twice a week with a vernier caliper and calculated as tumor volume (mm³) = 0.5 × (tumor long diameter x tumor short diameter²). Relative tumor inhibition rate TGI (%): TGI%=(1-T/C) × 100%. T/C% was the relative tumor growth rate, i.e., the percentage value of the relative tumor volume or tumor weight of the treatment group and PBS control group at a certain time point. T and C were tumor volume (TV) or tumor weight (TW) at a specific time point for the treatment group and the PBS control group, respectively. All data were expressed as Mean ± SEM, and student's t-test was used to compare whether there was significant difference in tumor volume and tumor weight between the treatment group and the control group, p <0.05 showing significant difference.

As shown in FIGs. 16 and 17, in the humanized HEK293-hCLDN18.2 model, the bispecific antibodies 31905-44 AA showed significant anti-tumor effect, and the terminal mean tumor volume of each group (PBS, 0.6 mg/kg 31905-44 AA, 3 mg/kg 31905-44 AA) was respectively: 230.59 mm³, 96.98 mm³, 97.6 mm³; Tumor Growth Inhibition values (TGI) were 101.8% in the 0.6 mg/kg 31905-44 AA group and 101.2% in the 3 mg/kg 31905-44 AA group. There was no significant weight loss in NCG mice in each administration group, indicating that NCG mice were well tolerated to the anti-CD3-CLDN18.2 bispecific antibodies at this dose.

**Table 11 Groups of experiments and Regimens of administration**

| Group | Grouping | Dose (mg/kg) | Regimen of administration | Mode of administration |
|---|---|---|---|---|
| Group 1 | PBMC + Control (PBS) | 0 | BIW*3 | Intraperitoneal administration |
| Group 2 | PBMC + 31905-44 AA | 0.6 | BIW*3 | Intraperitoneal administration |
| Group 3 | PBMC + 31905-44 AA | 3 | BIW*3 | Intraperitoneal administration |

### Example 6 Pharmacokinetic experiments for anti-CD3-CLDN18.2 bispecific antibodies

Two naive Cynomolgus macaques (one female and one male) were used in the experiment, with free water intake. The bispecific antibodies were administered at a dose of 1 mg/kg via intravenous infusion over 60 min. Blood collection time points were Pre-dose, 1 h, 4 h, 6 h, 24 h, 2 d, 4 d, 7 d, 10 d, 14 d, 21 d, 28 d, 35 d, and 42 d. The whole blood was collected and allowed to stand for 0.5 h, then centrifuged to collect the serum (4000 rpm, 10 min, 4°C). Before centrifugation, the blood samples were placed at room temperature. After centrifugation, the serum was aliquoted and cryopreserved at -80°C.

The concentration of bispecific antibodies in serum were detected by ELISA assay, and the detection process was described as follows:
A 96-well plate was coated with human CD3 protein at a concentration of 1 µg/mL, 100 µL per well, and stored at 4°C overnight; the plate was washed 3 times with 200 µL PBST per well, and incubated at 37°C for 1 h with 300 µL blocking reagent 5% milk powder; the plate was washed with 200 µL PBST per well for 3 times, 100 µL standard, QC, and test sample were added, and the mixture was incubated at 37°C for 1 h; the plate was washed with 300 µL PBST per well for 6 times, diluted 1: 5000 with 100 µL Anti-Human IgG Fc (HRP), and the mixture was incubated at 37°C for 1 h; the plate was washed with 300 µL PBST per well for 6 times, 100 µL TMB was added, the plate was stood 10 min away from light, and 100 µL stop solution was added to stop the chromogenic reaction.

The absorbance at 450 nm was detected with a M5 plate reader from MD, and the data were processed using softmax software.

The concentration of bispecific antibody 31905-44AA in Cynomolgus macaques serum was calculated using Phoenix Winnolin 8.2 software to obtain the pharmacokinetic parameters as shown in Table 12 below.

**Table 12 Cynomolgus macaques serum concentration and PK parameters for bispecific antibody 31905-44AA**

| Time (h) | Cynomolgus macaques (Female) | Cynomolgus macaques (Male) | Mean | SD | SEM |
|---|---|---|---|---|---|
| 0 | BLQ | BLQ | | | |
| 1 | 23.026 | 22.576 | 22.80 | 3.18 | 2.25 |
| 4 | 15.951 | 21.153 | 18.55 | 3.68 | 2.60 |
| 6 | 14.767 | 16.241 | 15.50 | 1.04 | 0.74 |
| 24 | 7.118 | 7.692 | 7.41 | 0.41 | 0.29 |
| 48 | 4.303 | 5.149 | 4.73 | 0.60 | 0.42 |
| 96 | 2.363 | 2.476 | 2.42 | 0.08 | 0.06 |
| 168 | 1.308 | 1.359 | 1.33 | 0.04 | 0.03 |
| 240 | BLQ | BLQ | | | |
| 336 | BLQ | BLQ | | | |
| T1/2 (hr) | 60.01 | 57.91 | 59.46 | 2.19 | 1.55 |
| Tmax (hr) | 1 | 1 | 1 | 0.0 | 0.0 |
| Cmax (µg/mL) | 23.026 | 22.567 | 22.80 | 3.18 | 2.25 |
| AUC₀₋₃₃₆ₕ (day*µg/mL) | 726.872 | 804.828 | 765.85 | 55.12 | 38.98 |
| AUC_{0-∞} (day*µg/mL) | 841.993 | 918.330 | 880.16 | 53.98 | 38.17 |
| Vd (mL/kg) | 104.5 | 91.0 | 97.75 | 9.55 | 6.75 |
| Cl (mL/day/kg) | 1.19 | 1.09 | 1.14 | 0.07 | 0.05 |
| MRT (hr) | 45.78 | 44.83 | 45.31 | 0.67 | 0.48 |

The concentration units in the above table were µg/mL and BLQ was below the limit of detection.

The half-life of the bispecific antibody 31905-44AA in Cynomolgus macaques was 59.46 ± 7.0 h, the Cmax was 22.80 ± 2.25 µg/mL, and the AUC₀₋₃₃₆ₕ was 765.85 ± 38.98 day * µg/mL; therefore, the bispecific antibody was stable in Cynomolgus macaques without obvious off-target binding and had good pharmacokinetic properties.

The embodiments of the present disclosure described above are intended to be merely exemplary, and equivalents of numerous specific compounds, materials, and operations may be recognized or determined by one skilled in the art without undue experimentation. All such equivalents are intended to be within the scope of the present disclosure and are encompassed by the claims.

## Claims

1. A bispecific antibody comprising anti-CLDN18.2 binding domain and anti-CD3 binding domain, the first binding domain being capable of binding to a CLDN18.2 protein, the second binding domain being capable of binding to CD3.

2. The bispecific antibody according to claim 1, wherein the anti-CLDN18.2 binding domain comprises: a heavy chain variable region in which the sequences of three CDRs, i.e., HCDR1, HCDR2, and HCDR3, are as set forth in SEQ ID NO: 11, 12, and 13, respectively; and a light chain variable region in which sequences of three CDRs, i.e., LCDR1, LCDR2, and LCDR3, are as set forth in SEQ ID NO: 14, 15, and 16, respectively.

3. The bispecific antibody according to claim 1, wherein the anti-CD3 binding domain comprises: a heavy chain variable region in which the sequences of three CDRs, i.e., HCDR1, HCDR2, and HCDR3, are as set forth in SEQ ID NO: 17, 18, and 19, respectively; and a light chain variable region in which sequences of three CDRs, i.e., LCDR1, LCDR2, and LCDR3, are as set forth in SEQ ID NO: 20, 21, and 22, respectively.

4. The bispecific antibody according to any one of the preceding claims, wherein the anti-CLDN18.2 binding domain comprises a heavy chain variable region and a light chain variable region, the heavy chain variable region has at least 80% to 100% sequence identity to SEQ ID NO: 23; and the light chain variable region has at least 80% to 100% sequence identity to SEQ ID NO: 24.

5. The bispecific antibody according to any one of the preceding claims, wherein the anti-CD3 binding domain comprises a heavy chain variable region and a light chain variable region, the heavy chain variable region has at least 80% to 100% sequence identity to SEQ ID NO: 6; and the light chain variable region has at least 80% to 100% sequence identity to SEQ ID NO: 7.

6. The bispecific antibody according to any one of the preceding claims, wherein the binding domain comprises a Fab, Fv, scFv, F(ab')₂, a linear antibody, a single domain antibody, or a full-length antibody.

7. The bispecific antibody according to any one of the preceding claims, further comprising a heavy chain constant region and/or a light chain constant region, preferably the heavy chain constant region comprising a native Fc or a variant Fc.

8. The bispecific antibody according to any one of the preceding claims, wherein the anti-CLDN18.2 binding domain is a full-length antibody and the anti-CD3 binding domain is scFv.

9. The bispecific antibody according to any one of the preceding claims, wherein the anti-CLDN18.2 binding domain is a full-length antibody, the full-length antibody has a heavy chain sequence as set forth in SEQ ID NO: 1, a light chain sequence as set forth in SEQ ID NO: 5; the anti-CD3 binding domain is scFv, the scFv has a sequence as set forth in SEQ ID NO: 8.

10. The bispecific antibody according to any one of the preceding claims, having a structure of linking anti-CD3 scFv at the C-terminus of the heavy or light chain of the full-length anti-CLDN18.2 antibody.

11. The bispecific antibody according to any one of the preceding claims, having a structure of fusing the anti-CD3 scFv to the C-terminus of the two light chains of the full-length anti-CLDN18.2 antibody to form two homologous light chains and two homologous heavy chains, wherein the fused light chain has a sequence as set forth in in SEQ ID NO: 2,the heavy chain has a sequence as set forth in SEQ ID NO: 1.

12. The bispecific antibody according to any one of the preceding claims, having a structure of fusing the anti-CD3 scFv to the C-terminus of one heavy chain of the full-length anti-CLDN18.2 antibody to form two homologous light chains and two heterologous heavy chains, wherein the heavy chain containing the scFv has a sequence as set forth in SEQ ID NO: 3, the heavy chain without the scFv has a sequence as set forth in SEQ ID NO: 4, the light chain has a sequence as set forth in SEQ ID NO: 5.

13. A nucleic acid encoding a bispecific antibody according to any one of the preceding claims.

14. A recombinant vector comprising the nucleic acid according to claim 13.

15. A host cell comprising the recombinant vector according to claim 14 or comprising the nucleic acid according to claim 13.

16. The host cell according to claim 15, which is a prokaryotic cell, such as E. coli; or a eukaryotic cell, such as yeast or mammalian cells, such as CHO cells, HEK293 cells, HEK293E cells, or Expi293 cells.

17. A method of preparing a bispecific antibody, comprising culturing the host cell according to claim 15 or 16 under suitable conditions and purifying the expression product from the cell.

18. Use of the bispecific antibody according to any one of the preceding claims for the preparation of a drug specifically targeting CLDN18.2-expressing tumor cells; the CLDN18.2-expressing tumor comprises: gastric cancer, pancreatic cancer, esophageal cancer, lung cancer, ovarian cancer, colon cancer, rectal cancer, liver cancer, head and neck cancer, and gallbladder cancer and metastases thereof, the gastric cancer metastasis being such as Kuckenberg tumor.

19. A pharmaceutical composition comprising an effective amount of the bispecific antibody according to any one of claims 1-12, or comprising an effective amount of the nucleic acid according to claim 13, or comprising an effective amount of the recombinant vector according to claim 14, or comprising an effective amount of the host cell according to claim 15 or 16.

20. The pharmaceutical composition according to claim 19, further comprising a pharmaceutically acceptable carrier.

21. The pharmaceutical composition according to claim 19 or 20, further comprising one or more additional other therapeutic agents; the additional therapeutic agents comprise: cytotoxic agents, cytostatic agents, anti-angiogenic agents, anti-neoplastic agents, chemotherapeutic agents, radio therapeutic agents, targeted anti-cancer agents, biological response modifiers, cancer vaccines, cytokines, hormones, anti-metastatic agents, and immunotherapeutic agents.

22. A drug box or kit comprising a container, and the pharmaceutical composition according to any one of claims 19-21 in the container.

23. A method of inducing cell death in CLDN18.2-expressing cells, comprising contacting the cells with the pharmaceutical composition according to any one of claims 19-21.

24. The method according to claim 23, wherein the cells are cancer cells, preferably solid tumor cells; more preferably, the cells are selected from the group consisting of: gastric cancer cells, esophageal cancer cells, intestinal cancer cells, pancreatic cancer cells, nephroblastoma cells, lung cancer cells, ovarian cancer cells, colon cancer cells, rectal cancer cells, liver cancer cells, head and neck cancer cells, chronic myelogenous leukemia cells, and gallbladder cancer cells.

25. A method of treating a disease associated with expression of CLDN18.2 in a subject, comprising administering to a subject in need thereof the pharmaceutical composition according to any one of claims 19-21.

26. The method according to claim 25, wherein the disease is a tumor; preferably gastric cancer, esophageal cancer, intestinal cancer, pancreatic cancer, nephroblastoma, lung cancer, ovarian cancer, colon cancer, rectal cancer, liver cancer, head and neck cancer, chronic myelogenous leukemia, or gallbladder cancer.

27. The method according to claim 25 or 26, further comprising administering to the subject one or more additional therapeutic agents.

28. The method according to claim 27, the one or more additional therapeutic agents comprising: chemotherapeutic agents, cytotoxic agents, radio therapeutic agents, cancer vaccines, anti-neoplastic agents, targeted anti-cancer agents, anti-angiogenic agents, biological response modifiers, cytokines, hormones, anti-metastatic agents, and immunotherapeutic agents.
